(19) Europäisches Patentamt / European Patent Office / Office européen des brevets

(11) **EP 4 006 616 B1**

(12) **EUROPEAN PATENT SPECIFICATION**

(45) Date of publication and mention
of the grant of the patent:
**22.11.2023   Bulletin 2023/47**

(21) Application number: **20846732.4**

(22) Date of filing: **09.07.2020**

(51) International Patent Classification (IPC):
*G02B 23/24* (2006.01)     *G06T 19/00* (2011.01)
*A61B 1/00* (2006.01)     *A61B 1/045* (2006.01)
*H04N 7/18* (2006.01)

(52) Cooperative Patent Classification (CPC):
**A61B 1/009; A61B 5/062; G06T 19/006;
G06T 19/20; H04N 7/185;** A61B 2034/102;
A61B 2034/2051; A61B 2034/2061; G06T 2210/41;
G06T 2219/2021

(86) International application number:
**PCT/JP2020/026920**

(87) International publication number:
**WO 2021/020067 (04.02.2021 Gazette 2021/05)**

(54) **ENDOSCOPE SHAPE DISPLAY CONTROL DEVICE, METHOD OF OPERATING ENDOSCOPE SHAPE DISPLAY CONTROL DEVICE, AND PROGRAM FOR OPERATING ENDOSCOPE SHAPE DISPLAY CONTROL DEVICE**

VORRICHTUNG ZUR STEUERUNG DER ANZEIGE EINER ENDOSKOPFORM, VERFAHREN ZUM BETRIEB EINER VORRICHTUNG ZUR STEUERUNG DER ANZEIGE EINER ENDOSKOPFORM UND PROGRAMM ZUM BETRIEB EINER VORRICHTUNG ZUR STEUERUNG DER ANZEIGE EINER ENDOSKOPFORM

DISPOSITIF DE COMMANDE D'AFFICHAGE DE FORME D'ENDOSCOPE, PROCÉDÉ DE FONCTIONNEMENT DE DISPOSITIF DE COMMANDE D'AFFICHAGE DE FORME D'ENDOSCOPE ET PROGRAMME POUR FAIRE FONCTIONNER UN DISPOSITIF DE COMMANDE D'AFFICHAGE DE FORME D'ENDOSCOPE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **31.07.2019   JP 2019141387**

(43) Date of publication of application:
**01.06.2022   Bulletin 2022/22**

(73) Proprietor: **FUJIFILM Corporation
Tokyo 106-8620 (JP)**

(72) Inventor: **YUMBE, Hirona
Ashigarakami-gun, Kanagawa 258-8538 (JP)**

(74) Representative: **Hughes, Andrea Michelle
Dehns Germany
Theresienstraße 6-8
80333 München (DE)**

(56) References cited:
**WO-A1-2018/116573      JP-A- H08 542
JP-A- 2000 079 087      JP-A- 2000 079 087
JP-A- 2001 046 320      JP-A- 2001 046 320
JP-A- 2002 369 790      JP-A- 2004 239 998
JP-A- 2009 015 165      JP-A- 2014 021 660
US-A1- 2003 055 317**

**Description**

Technical Field

The present disclosure relates to an endoscope shape display control device, an operation method of an endoscope shape display control device, and an operation program of an endoscope shape display control device.

Background Art

An endoscope that observes a lower digestive tract, such as the large intestine, is known. The lower digestive tract is intricately bent, unlike an upper digestive tract, such as an esophagus, which extends relatively linearly. Therefore, the endoscope, which is inserted into the lower digestive tract of a subject, such as a patient, an insertion part of the endoscope is also intricately bent. In order to grasp a shape of the insertion part of the endoscope inserted into the subject, an endoscope shape detection device that detects the shape of the insertion part of the endoscope in the subject (for example, see JP2004-551A) is known.

The endoscope shape detection device disclosed in JP2004-551Ahas a function of acquiring information for imaging the shape of the insertion part in the subject by using magnetism and imaging the shape of the insertion part based on the acquired information. For example, a plurality of magnetic field generation elements are disposed at intervals in the insertion part of the endoscope. The endoscope shape detection device detects a magnetic field generated by each magnetic field generation element in the insertion part by a magnetic field detection element disposed outside the subject. Since intensity of the magnetic field of each detected magnetic field generation element represents a position of each part of the insertion part with respect to the magnetic field detection element outside the subject, the endoscope shape detection device disclosed in JP2004-551A uses information on the intensity of the magnetic field of each magnetic field generation element as the information for imaging the shape of the insertion part to generate an image showing the shape of the insertion part. Further, the generated image is displayed on a monitor and presented to a user, such as an operator (for example, a doctor).

SUMMARY OF INVENTION

Technical Problem

An imaging procedure in the endoscope shape detection device of JP2004-55 1A is performed as follows, for example. First, the endoscope shape detection device disclosed in JP2004-551A specifies the position of each part of the insertion part to generate a three-dimension model of the insertion part based on the information for imaging the shape of the insertion part (for example, the information on the intensity of the magnetic field described above). Then, a rendering process is performed on the three-dimension model of the insertion part to generate a two-dimension image to be displayed on the monitor. The two-dimension image is an image showing the shape of the insertion part as viewed from one viewpoint in which the three-dimension model of the insertion part can be optionally set, and in the following, is referred to as a shape display image.

In a case in which the insertion part of the endoscope is bent in a loop shape in the subject, different portions of the insertion part intersect with each other in the shape display image depending on the set viewpoint. JP2004-551A discloses that a shade line process or a hidden surface process is performed in a case of the rendering process in order to express front and rear of the intersection portions.

However, in the method of performing the shade line process or the hidden surface process disclosed in JP2004-55 1A, it may be difficult to visually recognize a front-rear relationship of the intersection portions, that is, an overlapping condition of the insertion parts. That is, the insertion part of the endoscope has an elongated tubular shape. In a case in which the entire shape display image is reduction-displayed, in addition to a length of the insertion part, a diameter of the insertion part becomes thin at the same ratio.

Then, depending on the reduction rate, the insertion part may become too thin and may be displayed in a linear shape. In such a case, in a case in which the insertion part is bent in a loop shape, it looks as though the two lines intersect with each other. Even in a case in which the linear portion is subjected to the shading process and the hidden surface process, the effect of improving the visibility of the overlapping condition of the insertion parts cannot be expected. Therefore, there is a problem that it is difficult for the user to instantaneously determine the front and rear of the intersection portion (in other words, the overlapping condition of the insertion parts), for example, it is difficult to visually recognize the overlapping condition of the insertion parts. Further document US 2003/055317 A1 discloses an enlarged display of an endoscope shape with reduced magnification for the diameter of the insertion tube.

The technology of the present disclosure is to provide an endoscope shape display control device, an operation method of an endoscope shape display control device, and a program of the endoscope shape display control device capable of displaying a shape display image in which an overlapping condition of an insertion part is easily visually recognized even in a case of reduction-displaying the shape display image in which a shape of the insertion part of an endoscope inserted in a subject is displayed.

Solution to Problem

The present invention is directed to an endoscope shape display device as set out in claim 1, a corresponding operation method of claim 3 and a corresponding operation program of claim 4. In particular the present invention relates to an endoscope shape display control device comprising at least one processor, in which the processor acquires information for imaging a shape of an insertion part of an endoscope inserted in a subject, generates a shape display image in which the shape of the insertion part is displayed based on the acquired information, and controls, in a case in which the shape display image to be displayed is reduced, a reduction rate that is a reduced size with respect to a preset reference size, and executes, in a case in which an entire shape display image is reduced by a first reduction rate, a first control of setting the reduction rate to a second reduction rate larger than the first reduction rate for a diameter of the insertion part in the shape display image.

In the endoscope shape display control device according to the aspect described above, the processor may set the reduction rate to the first reduction rate for a length of the insertion part, and may set the reduction rate to the second reduction rate for the diameter of the insertion part.

In addition, in the endoscope shape display control device according to the present invention described above, the processor executes, in a case in which the first reduction rate is equal to or larger than a preset threshold value, a second control of setting the first reduction rate and the second reduction rate to the same value, and executes, in a case in which the first reduction rate is smaller than the threshold value, the first control.

Advantageous Effects of Invention

According to the technology of the present disclosure, it is possible to provide an endoscope shape display control device, an operation method of an endoscope shape display control device, and a program of the endoscope shape display control device capable of displaying a shape display image in which an overlapping condition of an insertion part is easily visually recognized even in a case of reduction-displaying the shape display image in which a shape of the insertion part of an endoscope inserted in a subject is displayed.

BRIEF DESCRIPTION OF DRAWINGS

Fig. 1 is an explanatory diagram showing a state of an endoscopic examination using an endoscope system.
Fig. 2 is a schematic view showing an overall configuration of the endoscope system.
Fig. 3 is a block diagram showing an electric configuration of the endoscope system.
Fig. 4 is an explanatory diagram showing a state in which a plurality of detection coils detect a magnetic field generated by a plurality of generation coils.
Fig. 5 is an explanatory diagram showing an example of magnetic field measurement data.
Fig. 6 is an explanatory diagram for describing a determination process by a determination unit and a position detection process of each detection coil by a position detection unit.
Fig. 7 is an explanatory diagram showing an example of a shape detection process of an insertion part.
Fig. 8 is a functional block diagram of an image generation unit.
Fig. 9 is a flowchart for describing a flow of a generation process of a shape display image by the image generation unit in a case of the same magnification display.
Fig. 10 is a flowchart for describing a flow of a modeling process by the image generation unit.
Fig. 11 is an explanatory diagram showing an example of a 3D model generation process of the insertion part.
Fig. 12 is a diagram showing an example of the shape display image.
Fig. 13 is a functional block diagram of the image generation unit and an information storage unit.
Fig. 14 is a diagram for describing a content of a first control.
Fig. 15 is an explanatory diagram showing an example of the 3D model generation process of the insertion part.
Fig. 16 is a diagram showing an example of the shape display image.
Fig. 17 is a diagram showing an example of a shape display image in the related art.
Fig. 18 is a flowchart for describing a flow of the modeling process by the image generation unit.

DESCRIPTION OF EMBODIMENTS

[Overall Configuration of Endoscope System]

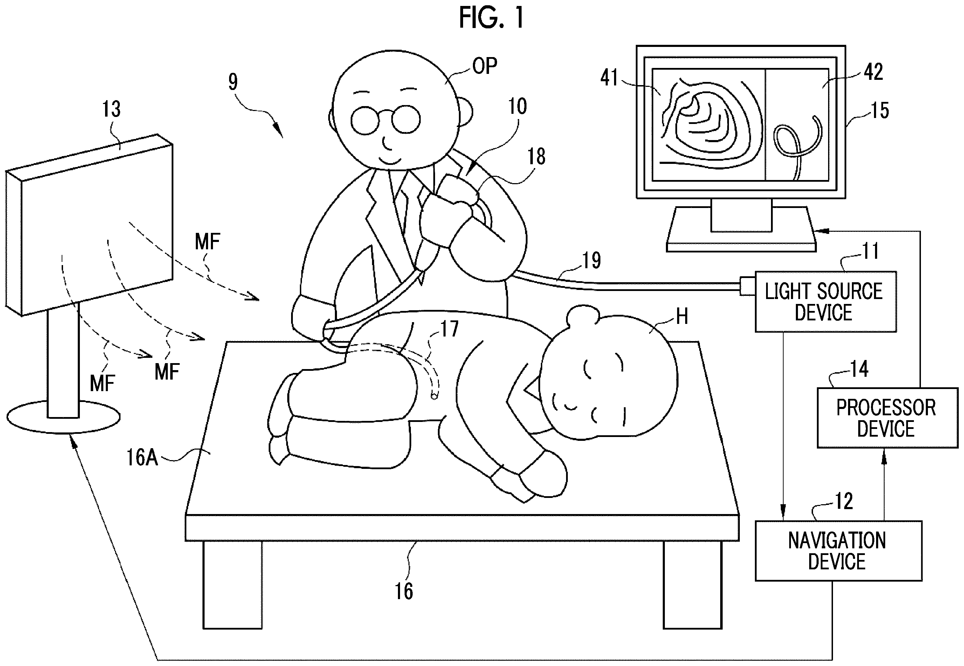

Fig. 1 is a schematic view showing an overall configuration of an endoscope system 9 comprising an endoscope shape display control device according to the technology of the present disclosure. As shown in Fig. 1, the endoscope system 9 comprises an endoscope 10, a light source device 11, a navigation device 12, a magnetic field generator 13, a processor device 14, and a monitor 15. The endoscope system 9 is used for an endoscopic examination in a body of a subject H, such as a patient. The subject H is an example of a subject. The endoscope 10 is, for example, an endoscope inserted into the digestive tract, such as the large intestine, and is a soft endoscope having flexibility. The endoscope 10 has an insertion part 17 to be inserted into the digestive tract, an operating part 18 installed consecutively to a proximal end side of the insertion part 17 and gripped by an operator OP to perform various operations, and a universal cord 19 installed consecutively to the operating part 18.

The endoscopic examination is performed, for example, in a state in which the subject H lies on an upper surface 16A of a bed 16. In a case of a large intestine examination, the insertion part 17 of the endoscope 10 is inserted into the digestive tract from an anus by an operator OP who is the doctor. The light source device 11 supplies illumination light that illuminates an inside of the large intestine, which is an observation site, to the endoscope 10. The processor device 14 generates an observation image 41 by processing an image captured by the endoscope 10. The observation image 41 is displayed on the monitor 15. The operator OP proceeds with the endoscopic examination while confirming the observation image 41. The observation image 41 displayed on the monitor 15 is basically a motion picture, but it is also possible to display a still picture as the observation image 41 as needed.

In addition, the endoscope system 9 has a navigation function of navigating technique, such as an insertion operation of the endoscope 10 performed by the operator OP. Here, the navigation means supporting the technique of the endoscope 10 of the operator OP by presenting an insertion state including the position and the shape of the insertion part 17 of the endoscope 10 in the body of the subject H to the operator OP. By the navigation function, the insertion state of the insertion part 17 is detected using a magnetic field MF and the detected insertion state is presented.

The navigation function is realized by the navigation device 12, the magnetic field generator 13, and a magnetic field measurement device in the endoscope 10 described below. The magnetic field generator 13 generates the magnetic field MF. The magnetic field generator 13 is disposed at a predetermined position within a range in which the generated magnetic field MF reaches the body of the subject H. The magnetic field generator 13 is an example of a magnetic field device according to the technology of the present disclosure.

The magnetic field measurement device in the endoscope 10 detects the magnetic field MF generated by the magnetic field generator 13 and measures the intensity of the detected magnetic field MF. The navigation device 12 detects the insertion state of the insertion part 17 by deriving a relative position between the magnetic field generator 13 and the insertion part 17 based on a magnetic field measurement result by the magnetic field measurement device. The processor device 14 generates a shape display image 42, which shows the insertion state detected by the navigation device 12.

The monitor 15 displays the observation image 41 and the shape display image 42. Note that the monitor 15 that displays the observation image 41 and the shape display image 42 may be provided separately.

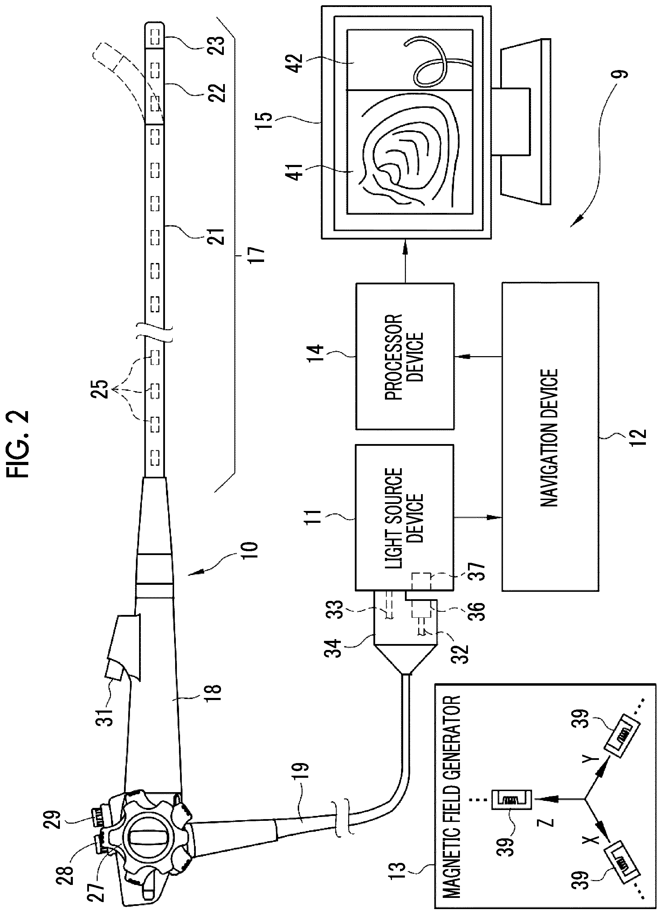

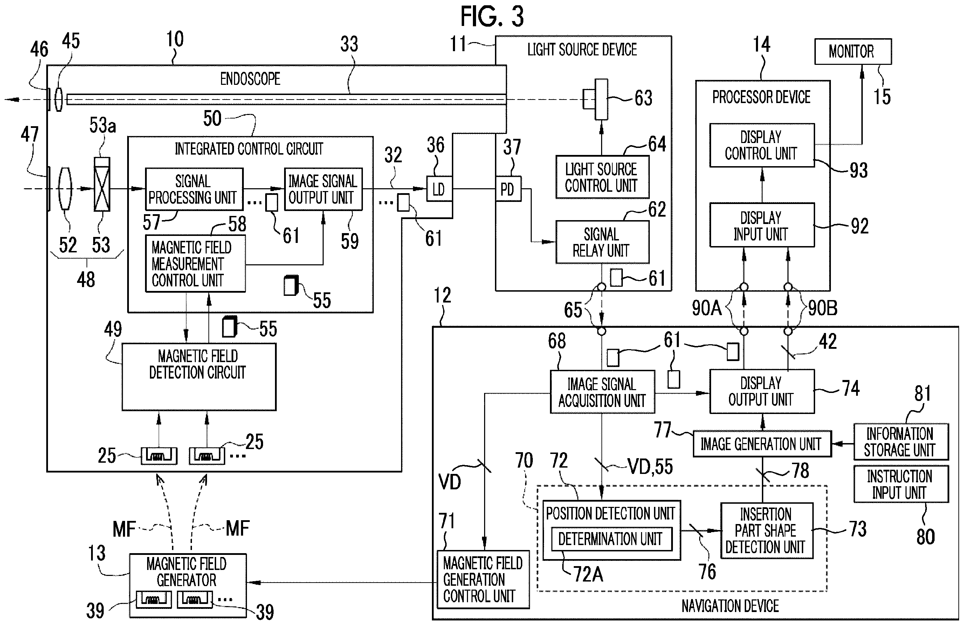

As shown in Fig. 2, the insertion part 17 is a tubular portion having a small diameter and a long length, and is configured by a soft part 21, a bendable part 22, and a distal end part 23, which are connected sequentially from the proximal end side to the distal end side. The soft part 21 has flexibility. The bendable part 22 is a part that can be bent by the operation of the operating part 18. An imaging apparatus 48 (see Fig. 3) and the like are disposed at the distal end part 23. In addition, although not shown in Fig. 2, an illumination window 46 (see Fig. 3) that illuminates the observation site with the illumination light, an observation window 47 (see Fig. 3) on which subject light of the illumination light reflected by the subject is incident, a treatment tool outlet (not shown) for a treatment tool to protrude, and a cleaning nozzle (not shown) for cleaning the observation window 47 by injecting the gas and the water into the observation window 47 are provided on a distal end surface of the distal end part 23.

A light guide 33, a signal cable 32, an operation wire (not shown), and a pipe line for inserting the treatment tool (not shown) are provided in the insertion part 17. The light guide 33 extends from the universal cord 19 and guides the illumination light supplied from the light source device 11 to the illumination window 46 at the distal end part 23. The signal cable 32 is used for power supply to the imaging apparatus 48 in addition to communication of an image signal from the imaging apparatus 48 and a control signal for controlling the imaging apparatus 48. Like the light guide 33, the signal cable 32 also extends from the universal cord 19 and is arranged to the distal end part 23.

The operation wire is a wire for operating the bendable part 22, and is arranged from the operating part 18 to the bendable part 22. The pipe line for inserting the treatment tool is a pipe line for inserting the treatment tool (not shown), such as forceps, and is arranged from the operating part 18 to the distal end part 23. In addition, a fluid tube for air/water supply is provided in the insertion part 17. The fluid tube supplies the distal end part 23 with the gas and the water for cleaning the observation window 47.

In addition, in the insertion part 17, a plurality of detection coils 25 are provided from the soft part 21 to the distal end part 23 at preset intervals. Each detection coil 25 corresponds to a magnetic field detection element which detects the magnetic field MF. Each detection coil 25 is affected by the magnetic field MF generated from the magnetic field generator 13, so that an induced electromotive force is generated by an operation of electromagnetic induction, and an induced current is generated by the induced electromotive force. A value of the induced current generated from each detection coil 25 represents the intensity of the magnetic field MF detected by each detection coil 25, which is the magnetic field measurement result. That is, the magnetic field measurement result refers to a value depending on the magnitude of the induced current, which represents the intensity of the magnetic field MF.

The operating part 18 is provided with various operation members operated by the operator OP. Specifically, the operating part 18 is provided with two types of bending operation knobs 27, an air/water supply button 28, and a suction button 29. Each of the two types of bending operation knobs 27 is connected to the operation wire, and is used for a right-left bending operation and an up-down bending operation of the bendable part 22. In addition, the operating part 18 is provided with a treatment tool inlet port 31 which is an inlet of the pipe line for inserting the treatment tool.

The universal cord 19 is a connection cord that connects the endoscope 10 to the light source device 11. The universal cord 19 encompasses the signal cable 32, the light guide 33, and the fluid tube (not shown). In addition, a connector 34 connected to the light source device 11 is provided at an end part of the universal cord 19.

By connecting the connector 34 to the light source device 11, the light source device 11 supplies the power, the control signal, the illumination light, the gas, and the water necessary for operating the endoscope 10 to the endoscope 10. In addition, the image signal of the observation site acquired by the imaging apparatus 48 (see Fig. 2) of the distal end part 23 and the magnetic field measurement result based on a detection signal of each detection coil 25 are transmitted from the endoscope 10 to the light source device 11.

The connector 34 is not electrically connected to the light source device 11 by wire using a metal signal line or the like, and instead, the connector 34 and the light source device 11 are connected to each other so as to be capable of optical communication (for example, non-contact type communication). The connector 34 transmits and receives the control signal exchanged between the endoscope 10 and the light source device 11 and transmits the image signal and the magnetic field measurement result from the endoscope 10 to the light source device 11 by optical communication. The connector 34 is provided with a laser diode (hereinafter, referred to as LD) 36 connected to the signal cable 32.

The LD 36 is used for transmitting a large amount of data from the endoscope 10 to the light source device 11, specifically, transmitting the image signal and the magnetic field measurement result. The LD 36 transmits the image signal and the magnetic field measurement result, which has been originally in a form of the electric signal, in a form of an optical signal to a photodiode (hereinafter, referred to as PD) 37 provided in the light source device 11.

Note that, although not shown, apart from the LD 36 and the PD 37, both the connector 34 and the light source device 11 are provided with a light transmission and reception unit that converts a small amount of the control signal exchanged between the endoscope 10 and the light source device 11 into the optical signal and transmits and receives the converted optical signal. Moreover, the connector 34 is provided with a power reception unit (not shown) that receives the power supplied from a power feed unit (not shown) of the light source device 11 by wireless power supply.

The light guide 33 in the connector 34 is inserted into the light source device 11. In addition, the fluid tube (not shown) in the connector 34 is connected to an air/water supply device (not shown) via the light source device 11. As a result, the light source device 11 and the air/water supply device supply the illumination light, the gas, and the water to the endoscope 10, respectively.

The light source device 11 supplies the illumination light to the light guide 33 of the endoscope 10 via the connector 34, and supplies the gas and the water supplied from the air/water supply device (not shown) to the fluid tube (not shown) of the endoscope 10. In addition, the light source device 11 receives the optical signal transmitted from the LD 36 by the PD 37, converts the received optical signal into the original image signal, which is the electric signal, and the magnetic field measurement result, and then outputs the converted image signal and the magnetic field measurement result to the navigation device 12.

The navigation device 12 outputs the image signal for generating the observation image 41 input from the light source device 11 to the processor device 14. In addition, the navigation device 12 controls the drive of the magnetic field generator 13 described below, detects the shape and the like of the insertion part 17 in the body of the subject H, and outputs a detection result to the processor device 14 as the information for generating the shape display image 42.

As described above, the endoscope 10 in the present embodiment is one-connector type having one connector 34 connected to the light source device 11. The endoscope 10 is connected to each of the processor device 14 and the navigation device 12 so as to be capable of communication via the light source device 11 to which the connector 34 is connected.

The magnetic field generator 13 has a plurality of generation coils 39 corresponding to a plurality of magnetic field generation elements. Each generation coil 39 includes, for example, an X-axis coil, a Y-axis coil, and a Z-axis coil that generate, by applying a drive current, an alternating current magnetic field (in other words, an alternating current magnetic field) in directions corresponding to XYZ coordinate axes of an orthogonal coordinate system XYZ, respectively. Each generation coil 39 generates the magnetic field MF having the same frequency. The generation coils 39 generate the magnetic fields MF at different timings from each other under a control of the navigation device 12, which will be described in detail below.

<Endoscope>

Fig. 3 is a block diagram showing an electric configuration of the endoscope system 9. As shown in Fig. 3, the endoscope 10 includes the light guide 33, an irradiation lens 45, the illumination window 46, the observation window 47, the imaging apparatus 48, a magnetic field detection circuit 49, an integrated control circuit 50, the signal cable 32, the LD 36, and the fluid tube and the cleaning nozzle (which are not shown).

The light guide 33 is, for example, a large-diameter optical fiber or a bundle fiber. An incident end of the light guide 33 is inserted into the light source device 11 via the connector 34. The light guide 33 is inserted into the connector 34, the universal cord 19, and the operating part 18, and an emission end faces the irradiation lens 45 provided in the distal end part 23 of the insertion part 17. As a result, the illumination light supplied from the light source device 11 to the incident end of the light guide 33 is emitted to the observation site from the irradiation lens 45 through the illumination window 46 provided on the distal end surface of the distal end part 23. Further, the illumination light reflected by the observation site is incident on an imaging surface of the imaging apparatus 48 as image light of the observation site through the observation window 47 provided on the distal end surface of the distal end part 23.

Note that one end side of the fluid tube (not shown) described above is connected to the air/water supply device (not shown) through the connector 34 and the light source device 11, and the other end side of the fluid tube (not shown) is connected to an air/water supply nozzle (not shown) provided on the distal end surface of the distal end part 23 through the insertion part 17 and the like. As a result, the gas or the water supplied from the air/water supply device (not shown) is injected into the observation window 47 from the air/water supply nozzle (not shown) to clean the observation window 47.

The imaging apparatus 48 includes a condenser lens 52 and an imaging element 53. The condenser lens 52 collects the image light of the observation site incident from the observation window 47, and forms the collected image light of the observation site on the imaging surface of the imaging element 53. The imaging element 53 is a complementary metal oxide semiconductor (CMOS) type or charge coupled device (CCD) type imaging element. The imaging element 53 is, for example, a color imaging element in which any of red (R), green (G), or blue (B) microfilter is assigned to each pixel. The imaging element 53 images the observation site, which is an observation target. More specifically, the imaging element 53 images the image light of the observation site imaged on the imaging surface (that is, converts the image light into the electric signal), and outputs the image signal of the observation site to the integrated control circuit 50.

In addition, the imaging element 53 is provided with an oscillation unit 53a that outputs a reference signal (for example, a clock signal), such as a crystal oscillator, and the imaging element 53 outputs the image signal constituting the motion picture with the reference signal oscillated from the oscillation unit 53a as a reference. An interval of the reference signal defines a frame rate. The frame rate is, for example, 30 frames per second (fps).

The magnetic field detection circuit 49 is electrically connected to each detection coil 25 in the insertion part 17. The magnetic field detection circuit 49 outputs magnetic field measurement data 55 including the magnetic field measurement result of each detection coil 25 depending on the magnetic field MF generated from the generation coil 39 of the magnetic field generator 13 to the integrated control circuit 50.

The integrated control circuit 50 configured to include arithmetic circuits including various central processing units (CPU) and various memories, and controls the operations of the units of the endoscope 10 in an integrated manner. The integrated control circuit 50 functions as a signal processing unit 57, a magnetic field measurement control unit 58, and an image signal output unit 59 by executing a control program stored in a memory (not shown). The magnetic field detection circuit 49 and the magnetic field measurement control unit 58 are combined to configure a magnetic field measurement unit. The magnetic field measurement unit measures a plurality of the magnetic fields MF originating from the generation coils 39 corresponding to the plurality of magnetic field generation elements based on the detection signals output by the detection coils 25, and outputs the magnetic field measurement result for each magnetic field MF. The magnetic field measurement unit and the detection coil 25 are combined to configure the magnetic field measurement device.

The signal processing unit 57 performs various signal processes on the image signals sequentially output from the imaging element 53. The signal process includes, for example, an analog signal process, such as a sampling two correlation pile process and a signal amplification process, and an analog/digital (A/D) conversion process of converting an analog signal into a digital signal after the analog signal process. The image signal after the signal process is performed is called a frame image signal 61. The signal processing unit 57 outputs the frame image signal 61 to the image signal output unit 59 depending on the frame rate. The frame image signal 61 is used as motion picture data of the observation site. As described above, a plurality of the frame image signals 61 are the image signals that are acquired by the imaging element 53 executing motion picture imaging and are output at preset time intervals.

The magnetic field measurement control unit 58 acquires the magnetic field measurement data 55 including a plurality of the magnetic field measurement results of the detection coils 25 via the magnetic field detection circuit 49, and outputs the acquired magnetic field measurement data 55 to the image signal output unit 59.

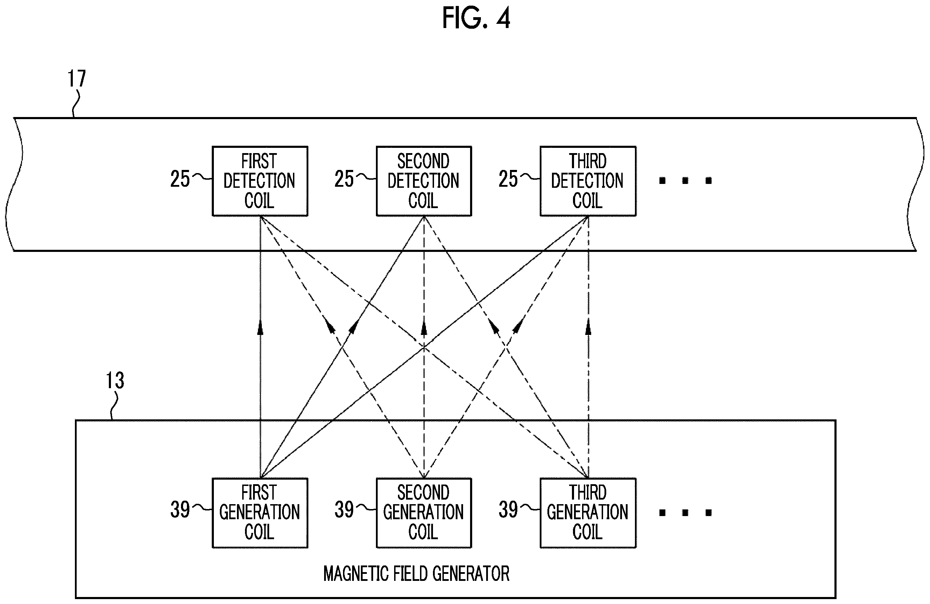

As shown in Fig. 4, even in a case in which the intensity of the magnetic field generated by each generation coil 39 is the same, for example, the magnetic field measurement result of each detection coil 25 is changed depending on a distance and a direction between each generation coil 39 that generates the magnetic field MF and each detection coil 25. For example, the first generation coil 39 shown in Fig. 4 has different distance and direction from each of the first to third detection coils 25, as shown by a solid line. Therefore, the magnetic field measurement results of the first to third detection coils 25 for the magnetic field MF generated by one first generation coil 39 are different. A relationship between each of the second generation coil 39 and the third generation coil 39 and each of the first to third detection coils 25 is the same.

In addition, on the contrary, even in a case in which the intensity of the magnetic field MF generated by each of the first to third generation coils 39 is the same, the magnetic field measurement result of one first detection coil 25 for the magnetic field MF of each generation coil 39 is different. Here, for example, a case is considered in which the first to third generation coils 39 are the X-axis coil, the Y-axis coil, and the Z-axis coil, respectively. In this case, a three-dimension coordinate position of the first detection coil 25 corresponding to the XYZ coordinate axes can be detected based on the magnetic field measurement result of one first detection coil 25 for the magnetic field MF of each of the X-axis coil, the Y-axis coil, and the Z-axis coil. The same applies to the second detection coil 25 and the third detection coil 25. In a case in which the three-dimension coordinate positions of the detection coils 25 provided in the insertion part 17 at preset intervals can be detected, the shape of the insertion part 17 can be detected.

Note that, actually, based on the magnetic field measurement result, an angle of each detection coil 25 is detected in addition to the three-dimension coordinate position of each detection coil 25. The shape of the insertion part 17 is detected based on the information of the three-dimension coordinate position and the angle. In the following, in order to avoid complication, the description regarding the angle will be omitted and only the three-dimension coordinate position will be described.

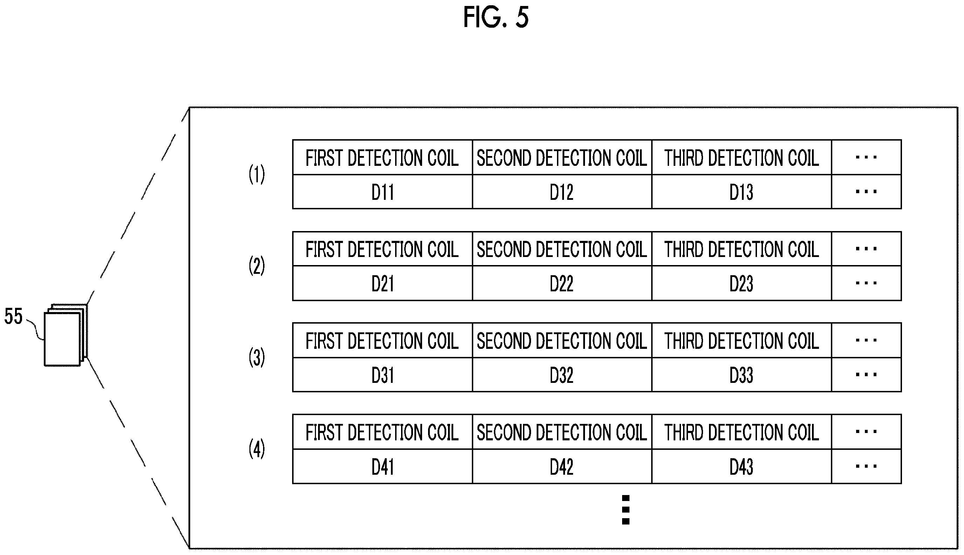

Fig. 5 is an explanatory diagram for describing an example of the magnetic field measurement data 55 acquired by the magnetic field measurement control unit 58. The magnetic field measurement control unit 58 detects the plurality of magnetic fields MF generated by the plurality of generation coils 39 by the plurality of detection coils 25 and acquires the magnetic field measurement data 55 including the plurality of magnetic field measurement results output from the detection coils 25.

In Fig. 5, (1) to (4) are data strings showing the magnetic field measurement results of the plurality of detection coils 25 with respect to the magnetic fields MF generated by the generation coils 39, respectively. For example, "D11" is a magnetic field measurement result in which the magnetic field MF generated by the first generation coil 39 is detected by the first detection coil 25. "D12" is a magnetic field measurement result in which the magnetic field MF generated by the first generation coil 39 is detected by the "second detection coil". Similarly, "D42" is a magnetic field measurement result in which the magnetic field MF generated by the fourth generation coil 39 is detected by the second detection coil 25. "D43" is a magnetic field measurement result in which the magnetic field MF generated by the fourth generation coil 39 is detected by the third detection coil 25.

The magnetic field measurement control unit 58 sequentially acquires the magnetic field measurement results of the detection coils 25 while synchronizing with a magnetic field generation timing of each generation coil 39 in the magnetic field generator 13. The magnetic field measurement control unit 58 acquires, for example, the magnetic field measurement results of all of the detection coils 25 for the magnetic fields MF of all of the generation coils 39 in one magnetic field measurement period defined by a synchronization signal described below. As a result, the magnetic field measurement control unit 58 acquires the magnetic field measurement data 55 including the plurality of magnetic field measurement results relating to all of combinations of the generation coils 39 and the detection coils 25 in one magnetic field measurement period.

For example, in a case in which 9 generation coils 39 are provided in the magnetic field generator 13 and 17 detection coils 25 are provided in the insertion part 17, 17 magnetic field measurement results are obtained for each generation coil 39. Therefore, the magnetic field measurement control unit 58 acquires the magnetic field measurement data 55 including a total of 9 × 17 = 153 magnetic field measurement results in one magnetic field measurement period. The magnetic field measurement data 55 including the plurality of magnetic field measurement results relating to all of such combinations is referred to as total magnetic field measurement data. In the present embodiment, unless otherwise specified, the magnetic field measurement data 55 shall include the total magnetic field measurement data.

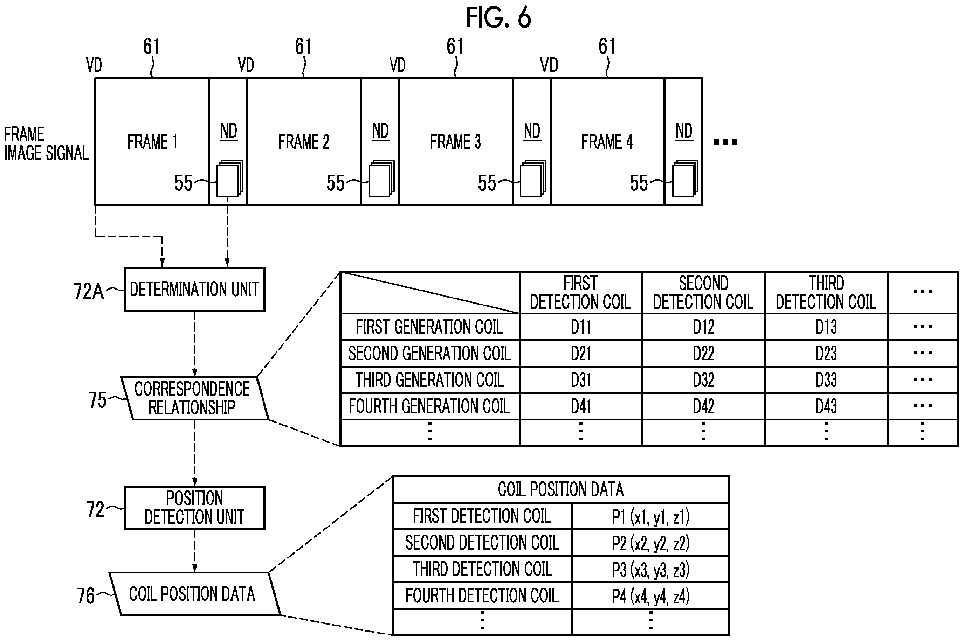

As shown in Fig. 6, the image signal output unit 59 adds a frame start signal VD to each of the plurality of frame image signals 61 sequentially input from the signal processing unit 57, and outputs the frame image signal 61. In Fig. 6, "frame 1", "frame 2", "frame 3" ... are frame numbers indicating output order of the plurality of frame image signals 61, which are shown for convenience. The frame start signal VD is a vertical synchronization signal, for example.

Moreover, the image signal output unit 59 adds the magnetic field measurement data 55 to the frame image signal 61 and outputs the frame image signal 61. That is, the frame start signal VD and the magnetic field measurement data 55 are included in all of the frame image signals 61 output by the image signal output unit 59. As shown in Fig. 6, the magnetic field measurement data 55 is added to a signal invalid region ND between the frame image signals 61, which corresponds to a blanking time of the imaging element 53. The blanking time is a vertical blanking period, for example. As described above, the frame start signal VD is also the signal included in the vertical blanking period of the plurality of frame image signals 61.

In Fig. 3, the image signal output unit 59 outputs the frame image signal 61 to the LD 36 described above via the signal cable 32. The LD 36 transmits the optical signal obtained by converting the frame image signal 61 into the optical signal to the PD 37 of the light source device 11.

As described above, the image signal output unit 59 outputs the frame image signal 61 acquired from the imaging element 53 via the signal processing unit 57 to the outside of the endoscope 10. In addition, by using the frame start signal VD included in the frame image signal 61 as the synchronization signal, the image signal output unit 59 functions as a synchronization signal generation unit.

<Light Source Device>

The light source device 11 includes an illumination light source 63, the PD 37, a light source control unit 64, a signal relay unit 62, and a communication interface 65. The illumination light source 63 is configured to include a semiconductor light source, such as the laser diode (LD) or a light emitting diode (LED), and is a white light source which emits white light having a wavelength range from a red region to a blue region as the illumination light. Note that, as the illumination light source 63, in addition to the white light source, a special light source that emits special light, such as purple light and infrared light, may be used. The illumination light emitted from the illumination light source 63 is incident on the incident end of the light guide 33 described above.

The light source control unit 64 is configured to include various arithmetic circuits including the CPU and various memories, and controls the operation of each unit of the light source device 11, such as the illumination light source 63.

The PD 37 receives the optical signal transmitted from the LD 36. The PD 37 converts the frame image signal 61 received in the form of the optical signal into the form of the original electric signal, and inputs the converted frame image signal 61 to the signal relay unit 62. The signal relay unit 62 is configured by, for example, a field programmable gate array (FPGA). The FPGA is a processor of which a circuit configuration can be changed after manufacturing, and the circuit configuration includes various arithmetic circuits and various memory circuits.

<Navigation Device>

The navigation device 12 includes an image signal acquisition unit 68, an insertion state detection unit 70, a magnetic field generation control unit 71, a display output unit 74, an image generation unit 77, an instruction input unit 80, and an information storage unit 81. Each unit of the navigation device 12 is configured by various arithmetic circuits (not shown) including one or a plurality of CPUs, and is operated by executing a control program stored in a memory (not shown).

The image signal acquisition unit 68 acquires the frame image signal 61 from the signal relay unit 62 via the communication interface 65. Further, the image signal acquisition unit 68 outputs the acquired frame image signal 61 to the display output unit 74.

In addition, the image signal acquisition unit 68 extracts the frame start signal VD and the magnetic field measurement data 55 included in the frame image signal 61, and outputs the extracted frame start signal VD and the magnetic field measurement data 55 to the insertion state detection unit 70. In addition, the image signal acquisition unit 68 extracts the frame start signal VD from the frame image signal 61, and outputs the extracted frame start signal VD to the magnetic field generation control unit 71.

The insertion state detection unit 70 detects the insertion state of the insertion part 17 of the endoscope 10 inserted into the body of the subject H based on the frame start signal VD and the magnetic field measurement data 55 acquired from the image signal acquisition unit 68. The insertion state detection unit 70 includes a position detection unit 72 and an insertion part shape detection unit 73.

The position detection unit 72 detects a position of each detection coil 25 based on the frame start signal VD and the magnetic field measurement data 55. A determination unit 72A is provided in the position detection unit 72.

As shown in Fig. 6, the determination unit 72A determines the plurality of magnetic field measurement results included in the magnetic field measurement data 55 with reference to a correspondence relationship 75. The correspondence relationship 75 is information indicating storage order of the plurality of magnetic field measurement results corresponding to a plurality of combinations of the generation coils 39 and the detection coils 25 included in the magnetic field measurement data 55. The determination unit 72A determines which combination of each generation coil 39 and each detection coil 25 corresponds to each magnetic field measurement result included in the magnetic field measurement data 55 based on the correspondence relationship 75.

Specifically, in the magnetic field measurement, generation order in which the generation coils 39 generate the magnetic field MF with the frame start signal VD as a reference and acquisition order of the magnetic field measurement results of the detection coils 25 for the magnetic field MF of one generation coil 39 are decided. The plurality of magnetic field measurement results corresponding to the combinations of the generation coils 39 and the detection coils 25 are stored in the magnetic field measurement data 55 depending on the generation order and the acquisition order. Therefore, the determination unit 72A can determine which combination of the plurality of magnetic field measurement results included in the magnetic field measurement data 55 (for example, "D11", "D12", "D13", ...) corresponds to each magnetic field measurement result by referring to the correspondence relationship 75 that defines the storage order with the frame start signal VD as a reference.

The position detection unit 72 detects, as coil position data 76, the position of each detection coil 25, specifically, the three-dimension coordinate position based on the plurality of magnetic field measurement results determined by the determination unit 72A. The coil position data is a relative position with the magnetic field generator 13 as a reference. In Fig. 6, for example, P1 indicates the three-dimension coordinate position (here, x1, y1, and z1) of the first detection coil 25. The same applies to P2, P3, P4, and the like.

In Fig. 3, the position detection unit 72 outputs the coil position data 76 to the insertion part shape detection unit 73. The insertion part shape detection unit 73 detects the shape of the insertion part 17 in the body of the subject H based on the coil position data 76 input from the position detection unit 72.

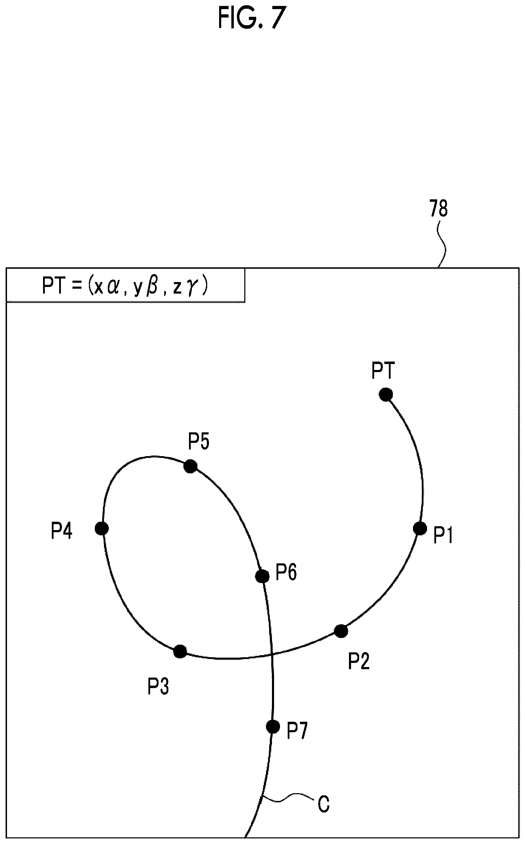

Fig. 7 is an explanatory diagram for describing an example of a shape detection process of the insertion part 17 by the insertion part shape detection unit 73. As shown in Fig. 7, the insertion part shape detection unit 73 performs an interpolation process of performing interpolation on each position with a curve based on the position (here, P1, P2, ...) of each detection coil 25 indicated by the coil position data 76, derives a central axis C of the insertion part 17, generates insertion part shape data 78 showing the shape of the insertion part 17. The interpolation process of performing interpolation with a curve is, for example, Bezier curve interpolation. The insertion part shape data 78 includes a distal end position PT of the distal end part 23 of the insertion part 17.

In Fig. 3, the insertion part shape detection unit 73 outputs the insertion part shape data 78 to the image generation unit 77. The insertion state detection unit 70 repeatedly performs a determination process by the determination unit 72A, a position detection process of detecting the coil position data 76 of each detection coil 25, a shape detection process of the insertion part 17, and an output of the insertion part shape data 78 each time the image signal acquisition unit 68 acquires new frame image signal 61.

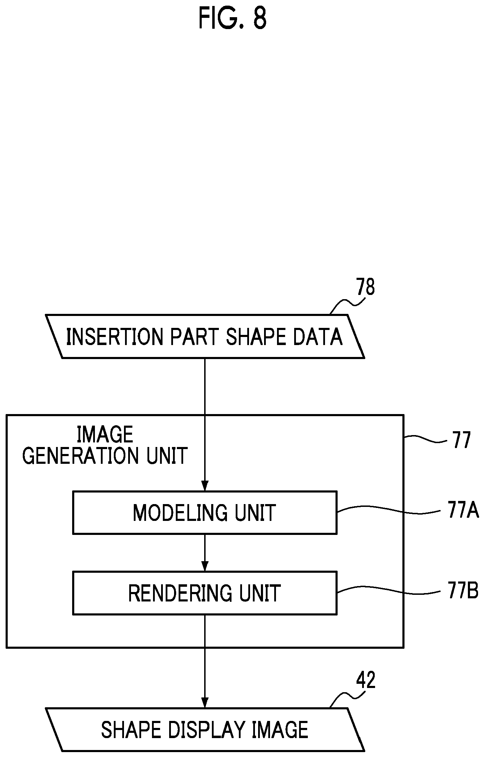

The image generation unit 77 generates the shape display image 42 based on the insertion part shape data 78. As shown in Fig. 8, a modeling unit 77A and a rendering unit 77B are provided in the image generation unit 77. As will be described in detail below, in the image generation unit 77, the modeling unit 77A generates a 3 dimension (D) model of the insertion part 17 based on the insertion part shape data 78, and the rendering unit 77B performs a rendering process on the 3D model of the insertion part 17, so that the 2 dimension (D) shape display image 42 showing the shape of the insertion part 17 is generated. The image generation unit 77 outputs data of the generated shape display image 42 to the processor device 14.

The image generation unit 77 updates the shape display image 42 each time new insertion part shape data 78 is input. Further, the image generation unit 77 outputs the updated data of the shape display image 42 to the processor device 14 each time the shape display image 42 is updated.

The instruction input unit 80 is an interface in a case in which the user uses the navigation device 12. The instruction input unit 80 is configured by, for example, a display comprising a touch panel that enables the user to perform a touch operation, an operation button, and the like. The instruction input unit 80 is provided such that an instruction for each unit of the navigation device 12, such as a display magnification change instruction, can be input.

The information storage unit 81 is configured by, for example, a non-volatile memory, such as a flash memory. The non-volatile memory holds the data stored in the memory even in a case in which a power source of the navigation device 12 is turned off.

The navigation device 12 is an example of an endoscope shape display control device according to the technology of the present disclosure that performs a display control of the shape display image 42. As described above, the magnetic field measurement data 55 is data obtained by using the plurality of detection coils 25 which are an example of a plurality of magnetic field detection elements according to the technology of the present disclosure provided in the insertion part 17 of the endoscope 10. The insertion part shape data 78 is information based on the magnetic field measurement data 55, and is an example of information according to the technology of the present disclosure for imaging the shape of the insertion part 17 of the endoscope 10 inserted in the subject H, which is an example of the subject. The image generation unit 77 generates the shape display image 42 obtained by imaging the shape of the insertion part 17 based on the insertion part shape data 78. In addition, the image generation unit 77 controls the reduction rate of the size of the shape display image 42 in a case in which the display magnification is changed. That is, the image generation unit 77 is an example of an information acquisition unit, an image generation unit, and a reduction rate control unit according to the technology of the present disclosure.

The display output unit 74 outputs the frame image signal 61 previously input from the image signal acquisition unit 68 described above and the data of the shape display image 42 input from the image generation unit 77 to the processor device 14 via communication interfaces 90A and 90B. In this case, the display output unit 74 associates the frame image signal 61 with the data of the shape display image 42 that corresponds in time with the frame image signal 61, and outputs the data to the processor device 14.

<Processor Device>

The processor device 14 has a display input unit 92 and a display control unit 93. The display input unit 92 sequentially outputs, to the display control unit 93, the data of the frame image signal 61 and the shape display image 42 sequentially input from the display output unit 74 via the communication interfaces 90A and 90B.

The display control unit 93 receives the input of the frame image signal 61 from the display input unit 92, and displays the observation image 41 (for example, the motion picture) based on the frame image signal 61 on the monitor 15 (see Figs. 1 and 2). In addition, the display control unit 93 receives input of the data of the shape display image 42 from the display input unit 92 and displays the shape display image 42 (for example, the motion picture) on the monitor 15 (see Figs. 1 and 2).

[Flow of Generation Process of Shape Display Image]

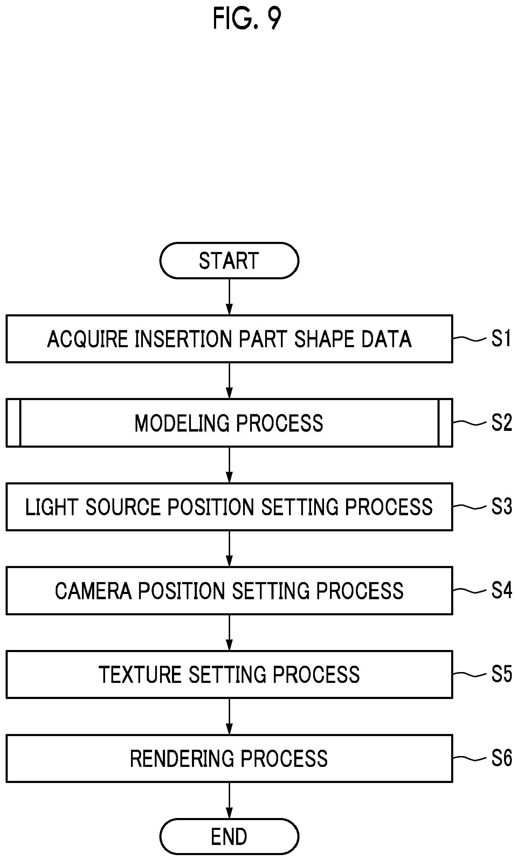

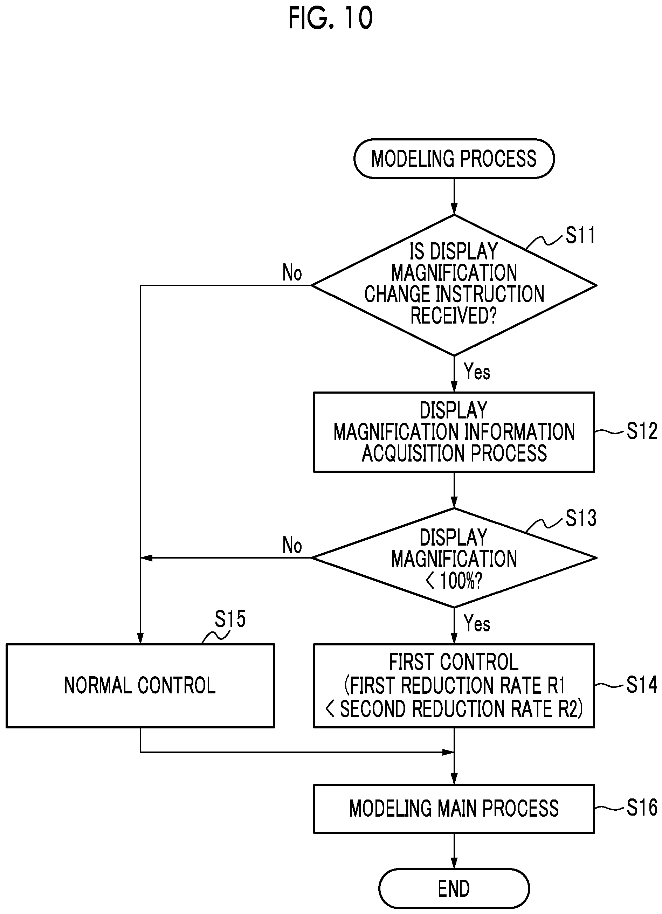

Fig. 9 is a flowchart for describing a flow of a generation process of the shape display image 42 by the image generation unit 77. Fig. 10 is a flowchart for describing a flow of a modeling process by the image generation unit 77. First, a case will be described in which the shape display image 42 is displayed at the same magnification.

As shown in the flowchart of Fig. 9, the image generation unit 77 executes an insertion part shape data acquisition process of acquiring the insertion part shape data 78 from the insertion state detection unit 70 (step S1). Next, in the image generation unit 77, the modeling unit 77A executes a modeling process of generating the 3D model of the insertion part 17 based on the insertion part shape data 78 (step S2).

As shown in the flowchart of Fig. 10, in the modeling process, first, the modeling unit 77A determines whether or not the display magnification change instruction is received (step S11).

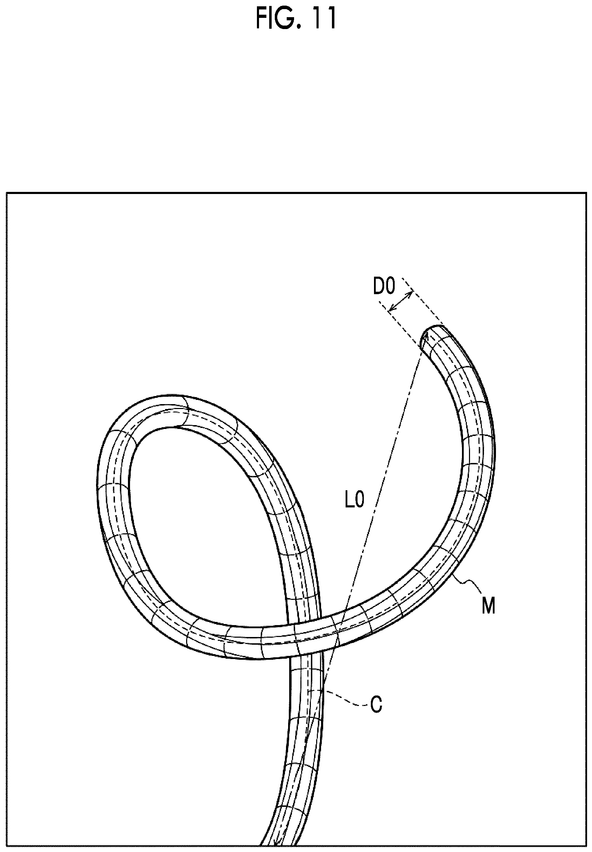

In a case in which it is determined in step S11 that the display magnification change instruction is not received (determination result No), as shown in Fig. 11, the modeling unit 77A executes a normal control of setting the display magnification of the entire shape display image 42 and the display magnification of the diameter of the insertion part 17 to the same value (step S15). In a case of the same magnification display, the display magnification of the entire shape display image 42 and the display magnification of the diameter of the insertion part 17 are both 100% (an example of a reference size).

Next, the modeling unit 77A executes a modeling main process of generating a cylindrical 3D model M having a preset reference diameter D0 with the central axis C of the insertion part 17 in the insertion part shape data 78 as the central axis (step S16). Here, a length of a preset portion of the 3D model M of the insertion part 17 is set as a reference length L0. Strictly speaking, the reference length L0 is the length of the preset portion of the central axis C of the insertion part 17, and is the length of the curve in a case in which the insertion part 17 is bent, but is shown by a straight line in Fig. 11 and the like for convenience.

Returning to the flowchart of Fig. 9, the rendering unit 77B executes a light source position setting process of setting the light source position in a case of rendering the 3D model M of the insertion part 17 (step S3). The light source position is set to a position with respect to the upper surface 16A of the bed 16 at which the light is emitted from an upper side in a virtual space in which the 3D model M is generated. Specifically, in a real space, in a case in which the magnetic field generator 13 and the bed 16 are installed on the same floor surface, a horizontal direction of the magnetic field generator 13 and the upper surface 16A of the bed 16 are in a parallel relationship. Therefore, in the virtual space, a plane parallel to the upper surface 16A of the bed 16 is set as an X-Y plane, and a Z-axis orthogonal to the X-Y plane is set. Further, in the virtual space, the light source position is set such that the light is emitted from an upper side of the set Z-axis to a lower side thereof.

Next, the rendering unit 77B executes a camera position setting process of setting a camera position (also referred to as a viewpoint position) in a case of rendering the 3D model M of the insertion part 17 (step S4). The camera position is set to a position with respect to the upper surface 16A of the bed 16 in the virtual space in which the 3D model M of the insertion part 17 is observed from the upper side of the Z-axis in the same manner as the light source position.

Next, the rendering unit 77B executes a texture setting process of finishing a surface of the 3D model M in a case of rendering the 3D model M of the insertion part 17 (step S5). In the texture setting process, the surface of the insertion part 17 is subjected to, for example, a process of making a texture of a single color plain pattern.

Finally, the rendering unit 77B adds a pattern to the surface of the 3D model M based on the set light source position, the camera, and the texture. In addition, the surface of the 3D model M added with the pattern is subjected to a shading process of performing shading corresponding to the light source. As described above, the rendering unit 77B executes the rendering process of generating the 2D shape display image 42 showing the shape of the insertion part 17 (step S6).

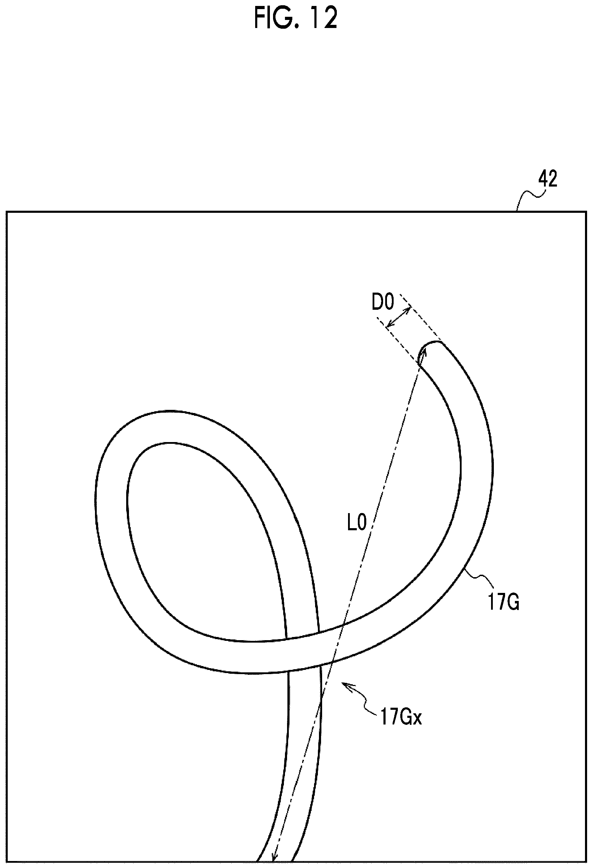

As a result, the shape display image 42 of the same magnification display is generated, as shown in Fig. 12. The shape display image 42 is configured to include an image showing the insertion part 17 and an image, which is a background thereof. In the following, the insertion part 17 in the shape display image 42, or more accurately, the image of the insertion part 17 in the shape display image 42, is simply referred to as the insertion part for simplification, and for distinguish from the actual insertion part 17, a reference numeral 17G is added and is referred to as an insertion part 17G

The generation process of the shape display image 42 is repeated each time the insertion part shape data 78 is updated.

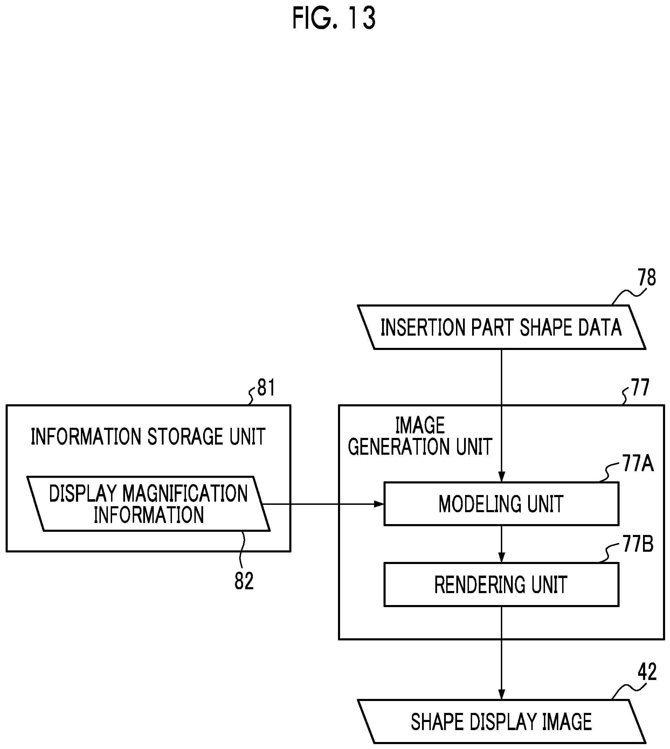

Next, a case will be described in which the display magnification change instruction of the shape display image 42 is given. Fig. 13 is a functional block diagram of the image generation unit 77 and the information storage unit 81.

The user inputs the display magnification change instruction via the instruction input unit 80. The display magnification of the entire shape display image 42 is included in the display magnification change instruction. The information indicating the display magnification is stored in the information storage unit 81 as display magnification information 82. The display magnification information 82 is updated to a content included in a new display magnification change instruction each time a new display magnification change instruction is given.

In a case in which the display magnification is 100%, the entire shape display image 42 is displayed at the same magnification (here, displayed at the reference size). In a case in which the display magnification exceeds 100%, the entire shape display image 42 is enlargement-displayed (here, enlargement-displayed with respect to the reference size). The display magnification in this case has the same meaning as an enlargement rate. In a case in which the display magnification is smaller than 100%, the entire shape display image 42 is reduction-displayed (here, reduction-displayed with respect to the reference size). The display magnification in this case has the same meaning as the reduction rate. The reduction rate corresponds to a first reduction rate R1 according to the technology of the present disclosure. In the following, the display magnification (here, the reduction rate) in a case in which the display magnification indicated by the display magnification information 82 is smaller than 100% will be described as the first reduction rate R1. The first reduction rate R1 is, for example, 50%.

Regarding the process in a case in which the display magnification change instruction is given, only a process content of the modeling process S2 in the flowchart of Fig. 9 is different. First, the insertion part shape data acquisition process (step S1) and the modeling setting process (step S2) of the flowchart of Fig. 9 are executed in order.

As shown in the flowchart of Fig. 10, in the modeling process, first, the modeling unit 77A determines whether or not the display magnification change instruction is received (step S11). In a case in which it is determined in step S11 that the display magnification change instruction is received (determination result Yes), the modeling unit 77A executes a display magnification information acquisition process of acquiring the display magnification information 82 from the information storage unit 81 (step S12).

Next, the modeling unit 77A determines whether or not the display magnification indicated by the display magnification information 82 is smaller than 100%, that is, whether or not it is reduction-displayed with respect to the shape display image 42 in a case of same magnification display (step S13). In a case in which it is determined in step S13 that the display magnification is smaller than 100% (that is, the shape display image 42 is reduction-displayed) (determination result Yes), the modeling unit 77A executes the first control of setting a second reduction rate R2, which is the reduction rate of the diameter of the insertion part 17 in the shape display image 42, to the value larger than the first reduction rate R1 (step S14).

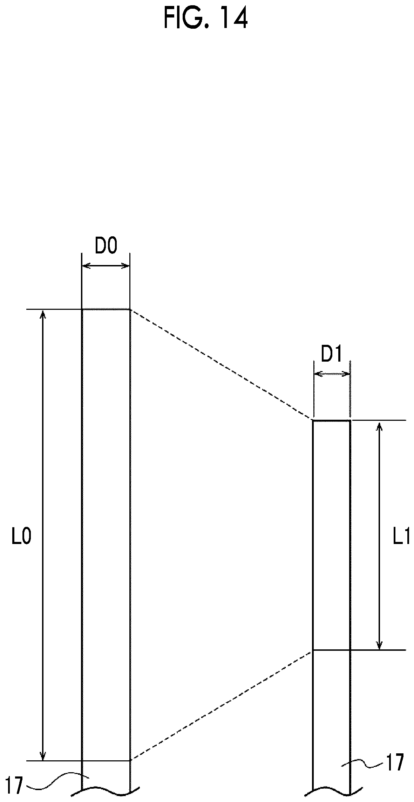

Fig. 14 is a diagram for describing a content of the first control executed by the modeling unit 77A. Note that, in Fig. 14, the insertion part 17 is displayed by the straight line in order to make the description easy to understand. The first reduction rate R1 is the reduction rate of the entire shape display image 42, and is the same value as a ratio (that is, L1/L0) of a reduced length L1 of the preset portion of the insertion part 17 of the reduced shape display image 42 to the reference length L0 of the preset portion of the insertion part 17 in a case of the same magnification display. The second reduction rate R2 is a ratio (that is, D1/D0) of a reduced diameter D1 of the insertion part 17 of the reduced shape display image 42 to the reference diameter D0 of the insertion part 17 in a case of the same magnification display. The first control is a control of setting the second reduction rate R2 to the value larger than the first reduction rate R1. As a result of the first control, the ratio of the reduced diameter D1 to the reduced length L1 is larger than the ratio of the reference diameter D0 to the reference length L0, so that the insertion part 17 is displayed to be thicker than the thickness corresponding to the first reduction rate R1.

Specifically, the modeling unit 77A calculates the second reduction rate R2 by, for example, the following expression. Here, the units of the first reduction rate R1 and the second reduction rate R2 are %. In a case in which the first reduction rate R1 is, for example, 50%, the second reduction rate R2 is set to 75%, which is the value larger than the first reduction rate R1 (here, 50%), as a result of calculation by the following expression.

$$R2 = \{(100 - R1)/2\} + R1$$

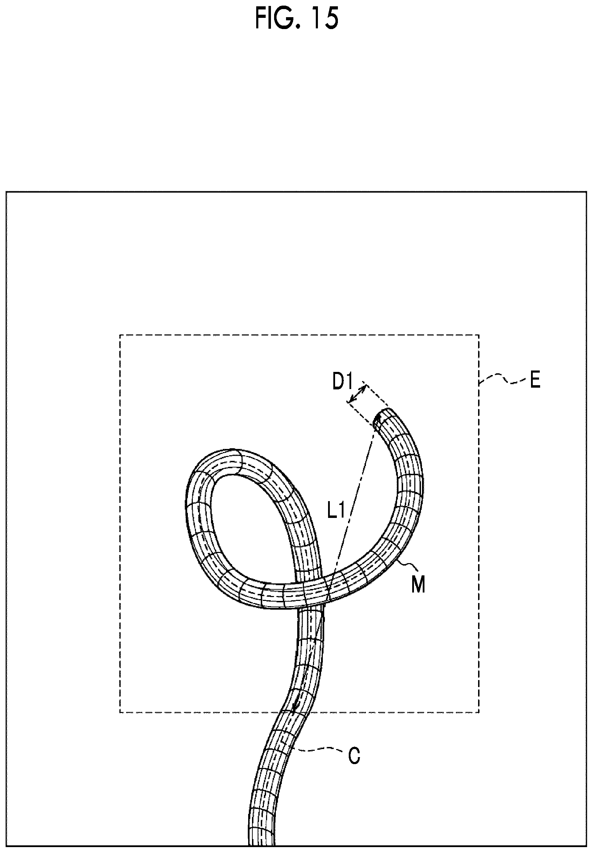

Fig. 15 is an explanatory diagram showing an example of a 3D model generation process of the insertion part in a case of the reduction-display. Note that Figs. 15 and 16 and 17 described below are explanatory diagrams and do not show accurate dimensions. As shown in Fig. 15, the modeling unit 77A executes the modeling main process of generating the cylindrical 3D model M with the central axis C of the insertion part 17 in the insertion part shape data 78 as the central axis (step S16). In the processes up to step S14, the first reduction rate R1, which is the reduction rate of the entire shape display image 42, is set to 50%, and the second reduction rate R2, which is the reduction rate of the diameter of the insertion part 17 in the shape display image 42, is set to 75%. Note that a region E shown by a dotted line in Fig. 15 indicates a display range in a case of the same magnification display.

Therefore, as shown in Fig. 15, the modeling unit 77A reduces the length of the entire insertion part 17 such that the reduced length L1 of the preset portion of the insertion part 17 of the reduced shape display image 42 becomes 50% of the reference length L0 of the preset portion of the insertion part 17 in a case of the same magnification display. In addition, the modeling unit 77A reduces the diameter of the insertion part 17 such that the reduced diameter D1 of the insertion part 17 becomes 75% of the reference diameter D0.

Returning to the flowchart of Fig. 9, the rendering unit 77B executes the light source position setting process (step S3), the camera position setting process (step S4), and the texture setting process (step S5) in this order. The rendering unit 77B executes a setting process having the same content as that in a case of the same magnification display in each setting process. In the end, the rendering unit 77B executes the rendering process (step S6).

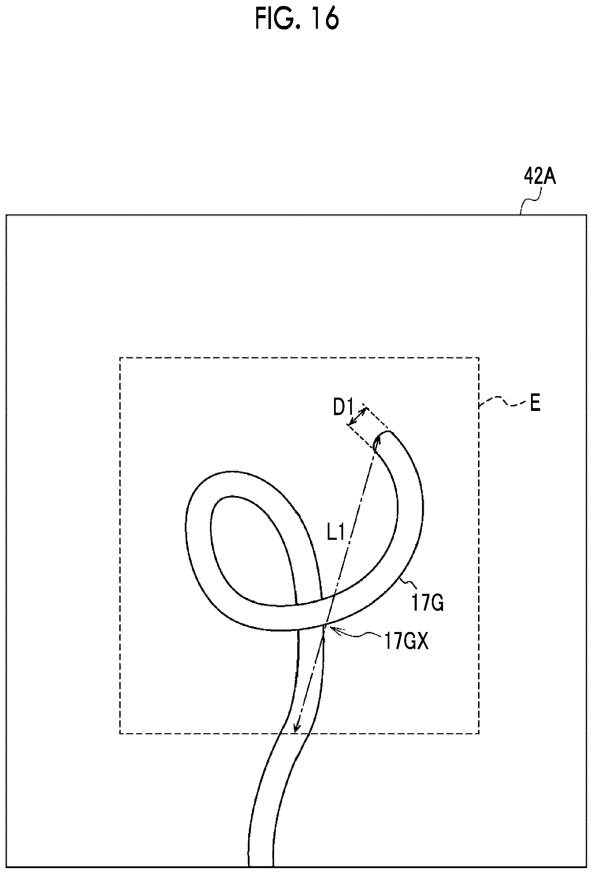

As a result, as shown in Fig. 16, a shape display image 42A obtained by reducing the entire shape display image 42 to 50% is generated. The shape display image 42A is configured to include an image showing the insertion part 17 and an image, which is a background thereof. In the following, the insertion part 17 in the shape display image 42A, or more accurately, the image of the insertion part 17 in the shape display image 42A, is simply referred to as the insertion part for simplification, and for distinguish from the actual insertion part 17, a reference numeral 17G is added and is referred to as an insertion part 17G

The generation process of the shape display image 42A is repeated each time the insertion part shape data 78 is updated.

Note that in a case in which the display magnification is equal to or larger than 100%, it is determined in step S13 of the flowchart of Fig. 10 that the display magnification is not smaller than 100% (that is, the entire shape display image 42 is displayed at the same magnification or enlargement-displayed) (determination result No). In this case, the modeling unit 77A executes the normal control for setting the display magnification of the entire shape display image 42 and the display magnification of the diameter of the insertion part 17 to the same value (step S 15), and the process proceeds to step S 16. As a result, the image in which the entire shape display image 42 is displayed at the same magnification or enlargement-displayed is acquired.

[Operation and Effect]

In the endoscope system 9 according to the present disclosure, as described above, in a case in which the shape display image 42 is reduction-displayed, the modeling unit 77A executes the first control of setting the second reduction rate R2, which is the reduction rate of the diameter of the insertion part 17 in the shape display image 42, to the value larger than the first reduction rate R1, which is the reduction rate of the entire shape display image 42.

As a result, as compared with the shape display image 42 of the same magnification display, in the shape display image 42A reduced to 50%, a reduced length GL1 is 50% of a reference length GL0, whereas a reduced diameter GD1 is 75% of a reference diameter GD0.

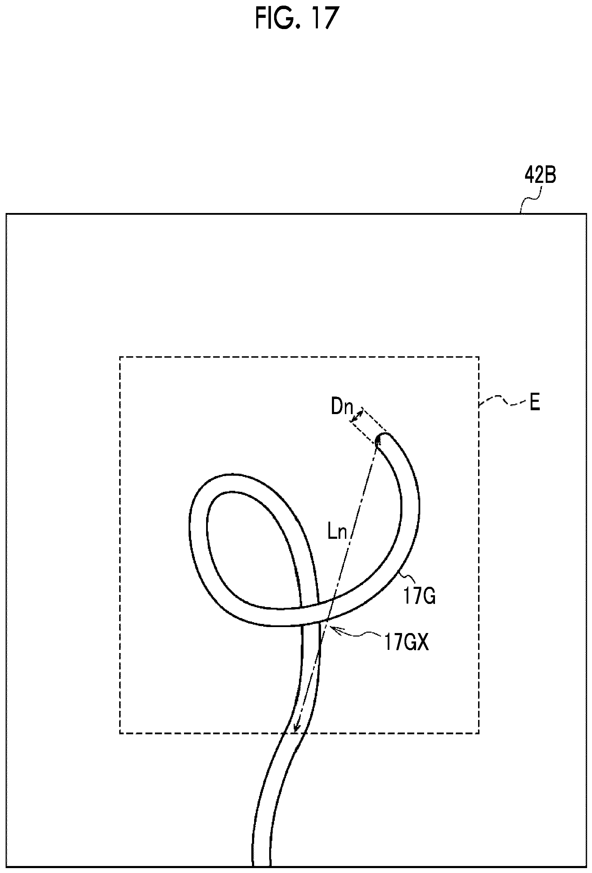

As a comparative example, Fig. 17 shows a shape display image 42B generated by the related art for uniformly reducing the entire shape display image 42. The shape display image 42B is an image obtained by uniformly reducing the entire display region by 50%. As compared with the shape display image 42 of the same magnification display, in the shape display image 42B reduced to 50%, a reduced length GLn is 50% of the reference length GL0, and a reduced diameter GDn is 50% of the reference diameter GD0.

In a case in which the shape display image 42 is reduced at the same reduction rate, the reduced diameter GD1 of the shape display image 42A generated based on the technology of the present disclosure is larger than the reduced diameter GDn of the shape display image 42B generated based on the related art. Therefore, it is possible to easily determine the front and rear of an intersection portion 17GX of the insertion part 17G As described above, according to the technology of the present disclosure, even in a case in which the shape display image 42 is reduction-displayed, it is possible to display the shape display image 42A in which the overlapping condition of the insertion parts 17G is easily visually recognized.

[Modification Example]

The embodiment described above is an example, and various modification examples are possible as shown below.

For example, in the embodiment described above, in a case in which the shape display image 42 is reduction-displayed, the modeling unit 77A always executes the first control of setting the second reduction rate R2, which is the reduction rate of the diameter of the insertion part 17 in the shape display image 42, to the value larger than the first reduction rate R1, which is the reduction rate of the entire shape display image 42. On the other hand, in a case in which the shape display image 42 is reduction-displayed, the modeling unit 77A may execute the first control only in a case in which the first reduction rate is smaller than a preset threshold value.

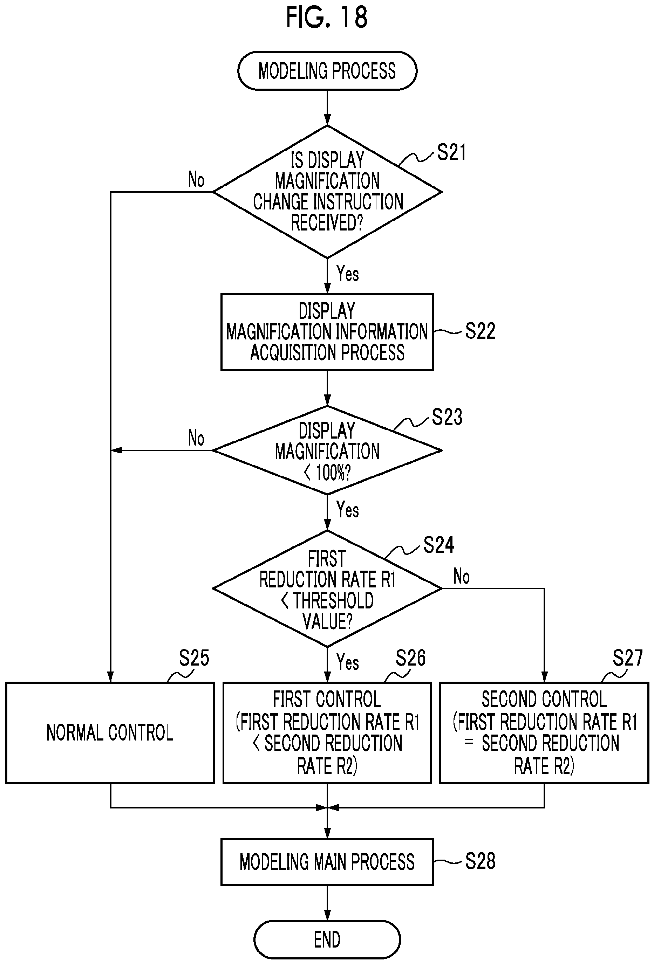

The flow of the process in this case will be described. The flow of the entire generation process of the shape display image 42 is as shown in the flowchart of Fig. 9 described above. In the present aspect, only the process content of the modeling process S2 in the flowchart of Fig. 9 is different. Fig. 18 is a flowchart for describing the flow of the modeling process of the present aspect. The threshold value can be set to any reduction rate.

As shown in the flowchart of Fig. 18, in the modeling process, first, the modeling unit 77A determines whether or not the display magnification change instruction is received (step S21). In a case in which it is determined in step S21 that the display magnification change instruction is received (determination result Yes), the modeling unit 77A executes the display magnification information acquisition process of acquiring the display magnification information 82 from the information storage unit 81 (step S22).

Next, the modeling unit 77A determines whether or not the display magnification indicated by the display magnification information 82 is smaller than 100%, that is, whether or not it is reduction-displayed with respect to the shape display image 42 in a case of same magnification display (step S23). In step S23, in a case in which it is determined that the display magnification is smaller than 100% (that is, the shape display image 42 is reduction-displayed) (determination result Yes), the modeling unit 77A determines whether or not the first reduction rate R1 is smaller than the threshold value (step S24).

In a case in which it is determined in step S24 that the first reduction rate R1 is smaller than the threshold value (determination result Yes), the modeling unit 77A executes the first control of setting a second reduction rate R2, which is the reduction rate of the diameter of the insertion part 17 in the shape display image 42, to the value larger than the first reduction rate R1 (step S26).

For example, in a case in which the first reduction rate R1 is 50% and the threshold value is 80%, the modeling unit 77A calculates the second reduction rate R2 by the following expression. Here, the units of the first reduction rate R1 and the second reduction rate R2 are %. The second reduction rate R2 is set to 65%, which is the value larger than the first reduction rate R1 (here, 50%), as a result of calculation by the following expression.

$$R2 = \{(80 - R1)/2\} + R1$$

In a case in which it is determined in step S24 that the first reduction rate R1 is not smaller than the threshold value (determination result No), the modeling unit 77A executes the second control of setting the second reduction rate R2, which is the reduction rate of the diameter of the insertion part 17 in the shape display image 42, to the same value as the first reduction rate R1 (step S27).

Next, the modeling unit 77A performs the modeling main process of generating the cylindrical 3D model M with the central axis C of the insertion part 17 in the insertion part shape data 78 as the central axis based on the first reduction rate R1 and the second reduction rate R2 (step S28), and the process proceeds to step S3 of the flowchart of Fig. 9.

Note that in a case in which it is determined in step S21 of the flowchart of Fig. 18 that the display magnification change instruction is not received (determination result No) and in a case in which it is determined in step S23 that the display magnification is not smaller than 100% (that is, the shape display image 42 is displayed at the same magnification or enlargement-displayed) (determination result No), the process proceeds to step S25. The modeling unit 77A executes the normal control for setting the display magnification of the entire shape display image 42 and the display magnification of the diameter of the insertion part 17 to the same value (step S25), and the process proceeds to step S28. As a result, the image in which the entire shape display image 42 is displayed at the same magnification or enlargement-displayed is acquired.

In a case in which the first control is executed in a case in which the shape display image 42 is reduction-displayed, the reduced diameter GD 1 of the insertion part 17G is displayed to be thicker than the actual insertion part 17 in the reduced shape display image 42A. However, in the present aspect, the second control is executed in a case in which the first reduction rate R1 is equal to or larger than the threshold value, and the first control is executed only in a case in which the first reduction rate R1 is smaller than the threshold value.

As a result, the diameter of the insertion part 17G can be displayed with the thickness corresponding to the first reduction rate R1 in a case in which the reduction rate is large and the insertion part 17 can be displayed without being too thin, and the diameter of the insertion part 17G can be displayed to be thicker than the thickness corresponding the first reduction rate R1 only in a case in which the reduction rate is small and the insertion part 17 is displayed to be thin. Therefore, it is possible to display a natural minified picture with less discomfort.

In addition, in the embodiment described above, a decision method of the second reduction rate R2 in the first control is not limited to the aspect of the decision using the expression described above, and any decision method may be adopted as long as the second reduction rate R2 is larger than the first reduction rate R1 in the decision method. For example, the decision may be made by using an expression different from the expression described above, or the decision may be made by referring to a table showing a relationship between the value of the first reduction rate R1 and the value of the second reduction rate R2.

In addition, in the embodiment described above, the process of generating the shape display image 42 after changing the display magnification is not limited to the method, which is described above, of changing the length and the diameter of the insertion part 17 in a case of modeling depending on the display magnification and fixing the camera position in a case of rendering, and any method may be adopted. For example, a method of fixing the length of the insertion part 17 in a case of modeling, changing only the diameter depending on the display magnification, and changing the camera position in a case of rendering depending on the display magnification may be adopted.

In addition, in the embodiment described above, the example has been described in which each generation coil 39 generates the magnetic field MF having the same frequency, but the frequencies of the magnetic fields MF generated by the generation coils 39 may be different.

In addition, in the embodiment described above, the example has been described in which the magnetic field measurement unit including the magnetic field measurement control unit 58 is disposed in the endoscope 10 and the magnetic field generation control unit 71 is disposed in the navigation device 12, on the contrary, the magnetic field generation control unit 71 may be disposed in the endoscope 10, and the magnetic field measurement unit may be disposed in the navigation device 12.

In addition, the embodiment described above, for example, as a hardware structure of the processing unit that executes various processes, such as the image generation unit 77 which is an example of the information acquisition unit, the image generation unit, and the reduction rate control unit various processors in the following can be used. The various processors include the central processing unit (CPU) that is a general-purpose processor executing the software and functioning as the various processing units, as well as a programmable logic device (PLD) that is a processor of which a circuit configuration can be changed after manufacture, such as a field programmable gate array (FPGA) and/or a dedicated electric circuit that is a processor having a circuit configuration that is designed for exclusive use in order to execute a specific process, such as an application specific integrated circuit (ASIC).

One processing unit may be configured by one of the various processors, or may be a combination of two or more processors of the same type or different types (for example, a combination of a plurality of FPGAs and/or a combination of a CPU and an FPGA). In this way, as the hardware structure, various processing units are configured by one or more of various processors described above.

Moreover, as the hardware structure of these various processors, more specifically, it is possible to use an electric circuit (circuitry) in which circuit elements such as semiconductor elements are combined.

An endoscope display control device having the information acquisition unit, the image generation unit, and the reduction rate control unit may be realized by the computer of which the processor is the CPU. A program for causing the CPU to function as the information acquisition unit and the image generation unit is an operation program of the endoscope display control device. The technology of the present disclosure extends to a computer-readable storage medium that stores the operation program non-transitorily, in addition to the operation program of the endoscope display control device.

In the present specification, "A and/or B" is synonymous with "at least one of A or B". That is, "A and/or B" means that it may be only A, only B, or a combination of A and B. In addition, in the present specification, in a case in which three or more matters are associated and expressed by "and/or", the same concept as "A and/or B" is applied.

The contents described and shown above are the detailed description of the parts relating to the technology of the present disclosure, and are merely an example of the technology of the present disclosure. For example, the above description of the configuration, the function, the action, and the effect are the description of examples of the configuration, the function, the action, and the effect of the parts relating to the technology of the present disclosure. Therefore, it is needless to say that unnecessary parts may be deleted, new elements may be added, or replacements may be made with respect to the contents described and shown above within the scope of the invention as defined by the claims.

## Claims

1. An endoscope shape display control device (12) comprising:

   at least one processor (14), wherein the processor
   acquires information for imaging a shape of an insertion part of an endoscope inserted in a subject,
   generates a shape display image in which the shape of the insertion part is displayed based on the acquired information, and
   controls, in a case in which the shape display image to be displayed is reduced, a reduction rate that is a reduced size with respect to a preset reference size, and executes, in a case in which an entire shape display image is reduced by a first reduction rate, a first control of setting the reduction rate to a second reduction rate larger than the first reduction rate for a diameter of the insertion part in the shape display image; and
   in a case in which the first reduction rate is equal to or larger than a preset threshold value, a second control of setting the first reduction rate and the second reduction rate to the same value, and executes, in a case in which the first reduction rate is smaller than the threshold value, the first control.

2. The endoscope shape display control device according to claim 1, wherein the processor sets the reduction rate to the first reduction rate for a length of the insertion part, and sets the reduction rate to the second reduction rate for the diameter of the insertion part.

**3.** An operation method of an endoscope shape display control device (12), the method comprising:

an information acquisition step of acquiring information for imaging a shape of an insertion part of an endoscope inserted in a subject; an image generation step of generating a shape display image in which the shape of the insertion part is displayed based on the acquired information; and a reduction rate control step of controlling, in a case in which the shape display image to be displayed is reduced, a reduction rate that is a reduced size with respect to a preset reference size and executing, in a case in which an entire shape display image is reduced by a first reduction rate, a first control of setting the reduction rate to a second reduction rate larger than the first reduction rate for a diameter of the insertion part in the shape display image; and in a case in which the first reduction rate is equal to or larger than a preset threshold value, a second control of setting the first reduction rate and the second reduction rate to the same value, and executes, in a case in which the first reduction rate is smaller than the threshold value, the first control.

**4.** An operation program of an endoscope shape display control device (12), the program causing a computer to function as:

an information acquisition unit that acquires information for imaging a shape of an insertion part of an endoscope inserted in a subject; an image generation unit that generates a shape display image in which the shape of the insertion part is displayed based on the acquired information; and a reduction rate control unit that controls, in a case in which the shape display image to be displayed is reduced, a reduction rate that is a reduced size with respect to a preset reference size and executes, in a case in which an entire shape display image is reduced by a first reduction rate, a first control of setting the reduction rate to a second reduction rate larger than the first reduction rate for a diameter of the insertion part in the shape display image; and in a case in which the first reduction rate is equal to or larger than a preset threshold value, a second control of setting the first reduction rate and the second reduction rate to the same value, and executes, in a case in which the first reduction rate is smaller than the threshold value, the first control.

**Patentansprüche**

**1.** Vorrichtung (12) zur Steuerung der Anzeige einer Endoskopform, umfassend:

mindestens einen Prozessor (14), wobei der Prozessor Informationen zum Abbilden einer Form eines Einführteils eines Endoskops, das in ein Subjekt eingeführt wird, erfasst, ein Formanzeigebild erzeugt, in dem die Form des Einführteils basierend auf den erfassten Informationen angezeigt wird, und in einem Fall, in dem das anzuzeigende Formanzeigebild verkleinert wird, eine Verkleinerungsrate steuert, die eine verkleinerte Größe in Bezug auf eine voreingestellte Referenzgröße darstellt, und in einem Fall, in dem ein gesamtes Formanzeigebild um eine erste Verkleinerungsrate verkleinert wird, eine erste Steuerung zum Einstellen der Verkleinerungsrate auf eine zweite Verkleinerungsrate ausführt, die größer als die erste Verkleinerungsrate für einen Durchmesser des Einführteils im Formanzeigebild ist; und in einem Fall, in dem die erste Verkleinerungsrate gleich oder größer als ein voreingestellter Schwellenwert ist, eine zweite Steuerung zum Einstellen der ersten Verkleinerungsrate und der zweiten Verkleinerungsrate auf den gleichen Wert, und in einem Fall, in dem die erste Verkleinerungsrate kleiner als der Schwellenwert ist, die erste Steuerung ausführt.

**2.** Vorrichtung zur Steuerung der Anzeige einer Endoskopform nach Anspruch 1, wobei der Prozessor die Verkleinerungsrate für eine Länge des Einführteils auf die erste Verkleinerungsrate einstellt und die Verkleinerungsrate für den Durchmesser des Einführteils auf die zweite Verkleinerungsrate einstellt.

**3.** Betriebsverfahren einer Vorrichtung (12) zur Steuerung der Anzeige einer Endoskopform, wobei das Verfahren Folgendes umfasst:

einen Informationserfassungsschritt zum Erfassen von Informationen zum Abbilden einer Form eines Einführteils eines Endoskops, das in ein Subjekt eingeführt wird; einen Bilderzeugungsschritt zum Erzeugen eines Formanzeigebilds, in dem die Form des Einführteils basierend auf den erfassten Informationen angezeigt wird; und

einen Verkleinerungsratensteuerungsschritt zum Steuern, in einem Fall, in dem das anzuzeigende Formanzeigebild verkleinert wird, einer Verkleinerungsrate, die eine verkleinerte Größe in Bezug auf eine voreingestellte Referenzgröße ist, und zum Ausführen, in einem Fall, in dem ein gesamtes Formanzeigebild um eine erste Verkleinerungsrate verkleinert wird, einer ersten Steuerung zum Einstellen der Verkleinerungsrate auf eine zweite Verkleinerungsrate, die größer als die erste Verkleinerungsrate für einen Durchmesser des Einführteils im Formanzeigebild ist; und

Ausführen, in einem Fall, in dem die erste Verkleinerungsrate gleich oder größer als ein voreingestellter Schwellenwert ist, einer zweiten Steuerung zum Einstellen der ersten Verkleinerungsrate und der zweiten Verkleinerungsrate auf den gleichen Wert, und in einem Fall, in dem die erste Verkleinerungsrate kleiner als der Schwellenwert ist, der ersten Steuerung.

4. Betriebsprogramm einer Vorrichtung (12) zur Steuerung der Anzeige einer Endoskopform, wobei das Programm einen Computer veranlasst, als Folgendes zu fungieren:

eine Informationserfassungseinheit, die Informationen zum Abbilden einer Form eines Einführungsteils eines Endoskops erfasst, das in ein Subjekt eingeführt wird;

eine Bilderzeugungseinheit, die basierend auf den erfassten Informationen ein Formanzeigebild erzeugt, in dem die Form des Einführteils angezeigt wird; und

eine Verkleinerungsratensteuereinheit, die in einem Fall, in dem das anzuzeigende Formanzeigebild verkleinert wird, eine Verkleinerungsrate steuert, die eine reduzierte Größe in Bezug auf eine voreingestellte Referenzgröße darstellt, und in einem Fall, in dem ein gesamtes Formanzeigebild um eine erste Verkleinerungsrate verkleinert wird, eine erste Steuerung zum Einstellen der Verkleinerungsrate auf eine zweite Verkleinerungsrate ausführt, die größer als die erste Verkleinerungsrate für einen Durchmesser des Einführteils im Formanzeigebild ist; und in einem Fall, in dem die erste Verkleinerungsrate gleich oder größer als ein voreingestellter Schwellenwert ist, eine zweite Steuerung zum Einstellen der ersten Verkleinerungsrate und der zweiten Verkleinerungsrate auf den gleichen Wert, und in einem Fall, in dem die erste Verkleinerungsrate kleiner als der Schwellenwert ist, die erste Steuerung ausführt.

**Revendications**

1. Dispositif de commande d'affichage de forme d'endoscope (12) comprenant :

au moins un processeur (14), dans lequel le processeur acquiert des informations pour imager une forme d'une partie d'insertion d'un endoscope inséré dans un sujet,
génère une image d'affichage de forme dans laquelle la forme de la partie d'insertion est affichée sur la base des informations acquises, et commande, dans un cas où l'image d'affichage de forme à afficher est réduite, un taux de réduction qui est une taille réduite par rapport à une taille de référence prédéfinie, et exécute, dans un cas où une image d'affichage de forme entière est réduite d'un premier taux de réduction, une première commande de réglage du taux de réduction à un second taux de réduction supérieur au premier taux de réduction pour un diamètre de la partie d'insertion dans l'image d'affichage de forme ; et
dans un cas où le premier taux de réduction est égal ou supérieur à une valeur seuil prédéfinie, une seconde commande de réglage du premier taux de réduction et du second taux de réduction à la même valeur, et exécute, dans un cas où le premier taux de réduction est inférieur à la valeur seuil, la première commande.

2. Dispositif de commande d'affichage de forme d'endoscope selon la revendication 1, dans lequel le processeur règle le taux de réduction au premier taux de réduction pour une longueur de la partie d'insertion, et règle le taux de réduction au second taux de réduction pour le diamètre de la partie d'insertion.

3. Procédé de fonctionnement d'un dispositif de commande d'affichage de forme d'endoscope (12), le procédé comprenant :

une étape d'acquisition d'informations consistant à acquérir des informations pour imager une forme d'une partie d'insertion d'un endoscope inséré dans un sujet ;
une étape de génération d'image consistant à générer une image d'affichage de forme dans laquelle la forme de la partie d'insertion est affichée sur la base des informations acquises ; et
une étape de commande de taux de réduction consistant à commander, dans un cas où l'image d'affichage de forme à afficher est réduite, un taux de réduction qui est une taille réduite par rapport à une taille de référence prédéfinie, et exécuter, dans un cas où une image d'affichage de forme entière est réduite d'un premier taux de réduction, une première commande de réglage du taux de réduction à un second taux de réduction supérieur au premier taux de réduction pour un diamètre de la partie d'insertion dans l'image d'affichage de forme ; et
dans un cas où le premier taux de réduction est égal ou supérieur à une valeur seuil prédéfinie, une seconde commande de réglage du premier taux de réduction et du second taux de réduction à la même valeur, et exécuter, dans un cas où le premier taux de réduction est inférieur à la valeur seuil, la première commande.

4. Programme de fonctionnement d'un dispositif de commande d'affichage de forme d'endoscope (12), le programme amenant un ordinateur à fonctionner en tant que :

une unité d'acquisition d'informations qui acquiert des informations pour imager une forme d'une partie d'insertion d'un endoscope inséré dans un sujet ;
une unité de génération d'image qui génère une image d'affichage de forme dans laquelle la forme de la partie d'insertion est affichée sur la base des informations acquises ; et
une unité de commande de taux de réduction qui commande, dans un cas où l'image d'affichage de forme à afficher est réduite, un taux de réduction qui est une taille réduite par rapport à une taille de référence prédéfinie, et exécute, dans un cas où une image d'affichage de forme entière est réduite d'un premier taux de réduction, une première commande de réglage du taux de réduction à un second taux de réduction supérieur au premier taux de réduction pour un diamètre de la partie d'insertion dans l'image d'affichage de forme ; et
dans un cas où le premier taux de réduction est égal ou supérieur à une valeur seuil prédéfinie, une seconde commande de réglage du premier taux de réduction et du second taux de réduction à la même valeur, et exécute, dans un cas où le premier taux de réduction est inférieur à la valeur seuil, la première commande.

FIG. 1

EP 4 006 616 B1

# FIG. 2

EP 4 006 616 B1

# FIG. 3

# FIG. 4

EP 4 006 616 B1

FIG. 5

55

|  | FIRST DETECTION COIL | SECOND DETECTION COIL | THIRD DETECTION COIL | · · · |
|---|---|---|---|---|
| (1) | D11 | D12 | D13 | · · · |

|  | FIRST DETECTION COIL | SECOND DETECTION COIL | THIRD DETECTION COIL | · · · |
|---|---|---|---|---|
| (2) | D21 | D22 | D23 | · · · |

|  | FIRST DETECTION COIL | SECOND DETECTION COIL | THIRD DETECTION COIL | · · · |
|---|---|---|---|---|
| (3) | D31 | D32 | D33 | · · · |

|  | FIRST DETECTION COIL | SECOND DETECTION COIL | THIRD DETECTION COIL | · · · |
|---|---|---|---|---|
| (4) | D41 | D42 | D43 | · · · |

## FIG. 6

FRAME IMAGE SIGNAL

| VD | FRAME 1 | ND 55 | VD | FRAME 2 | ND 55 | VD | FRAME 3 | ND 55 | VD | FRAME 4 | ND 55 | ... |

61  61  61  61

72A — DETERMINATION UNIT

75 — CORRESPONDENCE RELATIONSHIP

|  | FIRST DETECTION COIL | SECOND DETECTION COIL | THIRD DETECTION COIL | ... |
|---|---|---|---|---|
| FIRST GENERATION COIL | D11 | D12 | D13 | ... |
| SECOND GENERATION COIL | D21 | D22 | D23 | ... |
| THIRD GENERATION COIL | D31 | D32 | D33 | ... |
| FOURTH GENERATION COIL | D41 | D42 | D43 | ... |
| ⋮ | ⋮ | ⋮ | ⋮ | ⋮ |

72 — POSITION DETECTION UNIT

76 — COIL POSITION DATA

| COIL POSITION DATA | |
|---|---|
| FIRST DETECTION COIL | P1 (x1, y1, z1) |
| SECOND DETECTION COIL | P2 (x2, y2, z2) |
| THIRD DETECTION COIL | P3 (x3, y3, z3) |
| FOURTH DETECTION COIL | P4 (x4, y4, z4) |
| ⋮ | ⋮ |

EP 4 006 616 B1

FIG. 7

78

$PT = (x\alpha, y\beta, z\gamma)$

PT

P5

P4

P1

P6

P2

P3

P7

C

# FIG. 8

78

INSERTION PART SHAPE DATA

IMAGE
GENERATION UNIT — 77

MODELING UNIT — 77A

RENDERING UNIT — 77B

42

SHAPE DISPLAY IMAGE

# FIG. 9

```
                    ┌──────────────┐
                    │    START     │
                    └──────┬───────┘
                           │
                           ▼
        ┌──────────────────────────────────────┐
        │  ACQUIRE INSERTION PART SHAPE DATA    │─── S1
        └──────────────────┬───────────────────┘
                           │
                           ▼
        ┌┬─────────────────────────────────────┐
        ││        MODELING PROCESS             │─── S2
        └┴─────────────────┬───────────────────┘
                           │
                           ▼
        ┌──────────────────────────────────────┐
        │  LIGHT SOURCE POSITION SETTING PROCESS│─── S3
        └──────────────────┬───────────────────┘
                           │
                           ▼
        ┌──────────────────────────────────────┐
        │   CAMERA POSITION SETTING PROCESS     │─── S4
        └──────────────────┬───────────────────┘
                           │
                           ▼
        ┌──────────────────────────────────────┐
        │       TEXTURE SETTING PROCESS         │─── S5
        └──────────────────┬───────────────────┘
                           │
                           ▼
        ┌──────────────────────────────────────┐
        │        RENDERING PROCESS              │─── S6
        └──────────────────┬───────────────────┘
                           │
                           ▼
                    ┌──────────────┐
                    │     END      │
                    └──────────────┘
```

# FIG. 10

```
        ( MODELING PROCESS )
                  │
                  ▼
          ╱ IS DISPLAY ╲          S11
   No    ╱ MAGNIFICATION ╲
◄────────  CHANGE INSTRUCTION
         ╲ RECEIVED? ╱
          ╲       ╱
              │ Yes
              ▼
     ┌──────────────────────┐
     │       DISPLAY        │
     │ MAGNIFICATION INFORMATION │   S12
     │  ACQUISITION PROCESS  │
     └──────────────────────┘
              │
              ▼
          ╱ DISPLAY ╲          S13
   No    ╱ MAGNIFICATION ╲
◄────────    < 100%?
         ╲         ╱
          ╲       ╱
              │ Yes
```

S15

S14

┌──────────────────────┐        ┌──────────────────────┐
│                      │        │    FIRST CONTROL      │
│    NORMAL CONTROL    │        │ (FIRST REDUCTION RATE R1 │
│                      │        │ < SECOND REDUCTION RATE R2) │
└──────────────────────┘        └──────────────────────┘
              │                              │
              └──────────────────────────────┤
                                             ▼
                            ┌──────────────────────┐
                            │  MODELING MAIN PROCESS │   S16
                            └──────────────────────┘
                                             │
                                             ▼
                                        (   END   )

# FIG. 11

# FIG. 12

# FIG. 13

78

INSERTION PART SHAPE DATA

81

INFORMATION STORAGE UNIT

DISPLAY MAGNIFICATION INFORMATION

82

77

IMAGE GENERATION UNIT

77A

MODELING UNIT

77B

RENDERING UNIT

42

SHAPE DISPLAY IMAGE

EP 4 006 616 B1

FIG. 14

D0

D1

L0

L1

17

17

31

# FIG. 15

EP 4 006 616 B1

FIG. 17

42B

Dn

E

Ln

17G

17GX

34

## FIG. 18

```
        ( MODELING PROCESS )
                 │
                 ▼
        ╱───────────────────╲          S21
       ╱   IS DISPLAY         ╲
  No  ╱   MAGNIFICATION        ╲
◄─────   CHANGE INSTRUCTION
       ╲   RECEIVED?          ╱
        ╲───────────────────╱
                 │ Yes
                 ▼
        ┌───────────────────────┐
        │      DISPLAY          │
        │ MAGNIFICATION INFORMATION │  S22
        │  ACQUISITION PROCESS  │
        └───────────────────────┘
                 │
                 ▼
        ╱───────────────────╲          S23
       ╱      DISPLAY         ╲
  No  ╱    MAGNIFICATION       ╲
◄─────      < 100%?
       ╲                     ╱
        ╲───────────────────╱
                 │ Yes
                 ▼
        ╱───────────────────╲          S24
       ╱      FIRST           ╲
      ╱   REDUCTION RATE R1    ╲   No
      ╲   < THRESHOLD          ╱─────►
       ╲    VALUE?            ╱
        ╲───────────────────╱
                 │ Yes
```

S25

┌──────────────────┐
│                  │
│  NORMAL CONTROL  │
│                  │
└──────────────────┘

S26

┌──────────────────────────┐
│    FIRST CONTROL         │
│ (FIRST REDUCTION RATE R1 │
│  < SECOND REDUCTION      │
│    RATE R2)              │
└──────────────────────────┘

S27

┌──────────────────────────┐
│    SECOND CONTROL        │
│ (FIRST REDUCTION RATE R1 │
│  = SECOND REDUCTION      │
│    RATE R2)              │
└──────────────────────────┘

```
        ┌───────────────────────┐
        │  MODELING MAIN PROCESS │  S28
        └───────────────────────┘
                 │
                 ▼
             (  END  )
```

**EP 4 006 616 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004000551 A

- US 2003055317 A1